# FASCICULE DE BREVET EUROPEEN

(11) **EP 0 895 805 B1**
(45) Date de publication et mention de la délivrance du brevet: **01.10.2003**
(21) Numéro de dépôt: 98401809.3
(22) Date de dépôt: 17.07.1998
(51) Int. Cl.: B01F 17/00

(54) **Composition émulsionnante à base de polyglycosides et d'alcool gras**
Emulgierende Zusammensetzung aus Polyglycosiden und Fettalkohol
Emulsifying composition containing polyglycosides and fatty alcohol

(30) Priorité: 08.08.1997 FR 9710213
(43) Date de publication de la demande: 10.02.1999
(73) Titulaire: AGRO INDUSTRIE RECHERCHES ET DEVELOPPEMENTS (A.R.D.), 51110 Pomacle (FR)
(72) Inventeur: Bertho, Jean-Noel, 51110 Pomacle (FR); Mathaly, Philippe, 51100 Reims (FR); de Baynast, Régis, 78000 Versailles (FR); Dubois, Véronique, 51100 Reims (FR)
(74) Mandataire: Eidelsberg, Victor Albert

(56) Documents cités:
- WO-A-92/06778
- WO-A-95/13863
- WO-A-96/37285

## Description

La présente invention concerne une composition comprenant un mélange de polyglycosides et au moins un alcool gras, l'utilisation de ces compositions en tant que composition émulsionnante, composition auto-émulsionnable pour la préparation d'émulsions ainsi que des émulsions comprenant une telle composition.

Les émulsions sont largement produites et utilisées dans l'industrie soit comme matériaux à consommer, soit à appliquer sur des surfaces en tant que vecteurs d'agents non solubles dans l'eau. On retrouve des émulsions en cosmétique (laits, crèmes, pommades), en cuisine (sauces, crèmes), en galénique (pommades, crèmes), en peinture (peinture sans odeur), dans l'industrie routière (bitume en émulsion), en agrochimie (produits phytosanitaires), en détergence, dans le laminage, la sidérurgie et dans la fabrication de dépôts divers (imprimerie, adhésifs ...).

En cosmétique et en pharmacie, pour la conception de produits d'hygiène ou de soins, les émulsions constituent un moyen efficace pour obtenir la combinaison harmonieuse d'ingrédients de nature et de propriétés différentes en une présentation homogène et facile d'emploi. Les émulsionnants les plus utilisés à ce jour sont les sulfates et les sulfonates d'alkyle, les alcools, les acides, les esters gras éthoxylés, les esters gras de sorbitan...

De nombreux composés phytosanitaires sont insolubles dans l'eau et, étant préalablement solubilisés dans un solvant organique, ils pourront être émulsionnés dans l'eau au moment de l'application ou de la formulation par un choix approprié d'émulsionnants.

C'est dans les présentations liquides de produits phytosanitaires que l'on retrouve le plus d'agents émulsionnants. Le concentré émulsionnable qui est la forme la plus courante et encore la plus usitée sur le marché actuel contient classiquement 250 g/l de pesticides par exemple et 50 g/l d'émulsionnants. La mise en oeuvre correspond à la formation d'une émulsion fine dont la stabilité doit être assurée pendant plusieurs heures indépendamment de la température ou de la dureté de l'eau. Les suspensions concentrées sont de développement plus récent et correspondent à la génération des formulations permettant d'appliquer de très faibles doses à l'hectare. C'est le cas par exemple des émulsions concentrées qui contiennent respectivement de 400 à 600 g/l de pesticides et de 50 à 100 g/l d'émulsionnants. Contrairement aux deux présentations précédentes, les microémulsions sont des systèmes thermodynamiquement stables, donc très intéressantes sur le plan du stockage des produits.

Les émulsionnants utilisés pour le domaine phytosanitaire sont essentiellement, en anioniques, le dodécylbenzènesulfonate de calcium, les alkylarylsulfonatés d'amines, les phosphates esters d'alcool gras éthoxylés ou d'alkylphénols éthoxylés. En non ioniques, les plus utilisés sont les alkylphénols éthoxylés, les alcools et acides gras éthoxylés.

La préparation des émulsions au moyen de tensioactifs non ioniques polyoxyéthylénés est bien connue.

Ces tensioactifs, émulsionnants, peuvent se présenter sous la forme de compositions à base d'alcools gras, d'acides ou d'esters gras qui présentent l'avantage d'être "auto-émulsionnable". On entend par "auto-émulsionnable" une composition qui permet d'obtenir une émulsion stable par simple mélange sous agitation modérée avec une phase aqueuse.

Les tensioactifs non ioniques polyoxyéthylénés mentionnés ci-dessus présentent l'inconvénient, pour des applications notamment en cosmétique, dermatologie et en pharmacie, d'être parfois irritants et d'être susceptibles de contenir des impuretés indésirables liées à l'utilisation de l'oxyde d'éthylène, comme par exemple le 1-4 dioxanne.

Par ailleurs, il a pu être constaté que les émulsions préparées avec ces compositions auto-émulsionnables ne présentaient une stabilité que sur une période de temps relativement courte.

En vue de remédier à ces inconvénients, il a été proposé dans la demande WO-92/06778 l'utilisation de compositions auto-émulsionnables à base d'alcools gras et de polyglycosides ou polyosides d'alkyle. Celles-ci comportent, de préférence, un mélange de polyglycosides d'alkyle dont les chaînes grasses comprennent 16 et 18 atomes de carbone, ainsi qu'un mélange d'alcools gras de même longueur de chaîne grasse.

Cependant, il a été constaté d'une part que l'utilisation de la composition auto-émulsionnable selon WO-92/06778, ne permettait pas toujours d'obtenir des émulsions suffisamment stables, notamment pour des concentrations en compositions inférieures à 5 % en poids par rapport au poids total de l'émulsion.

D'une part, il a été constaté dans la demande EP 0628305, que l'utilisation de la composition auto-émulsionnable selon WO 92/06778, ne permettait également pas d'obtenir des émulsions suffisamment stables dans le temps en présence d'huile végétales particulièrement riches en acide linoléique telles que l'huile de tournesol, l'huile de germes de blé, l'huile de soja, l'huile de pépins de raisins...

Cet acide linoléique a cependant un rôle très important dans le maintien des structures lipidiques des espaces intercellulaires du stratum corneum ainsi que dans la restauration de la fonction de barrière des peaux sèches.

La demanderesse a également constaté que les huiles de silicones se prêtent mal elles aussi à l'obtention d'émulsions stables. Ces huiles sont cependant intéressantes, puisqu'elles permettent d'obtenir des émulsions au toucher agréable, présentant une bonne pénétration et généralement résistantes à l'eau.

Il est alors proposé, dans la présente invention, la préparation de compositions à base de polyglycosides et d'alcools gras, permettant l'obtention d'émulsions stables (c'est-à-dire ne présentant pas de déphasage après trois mois de vieillissement à 45° C) même en utilisant moins de 5 % en poids de composition à base de polyglycosides de l'invention par rapport au poids total de l'émulsion, même en présence d'huiles végétales riches en acide linoléique ou d'huiles silicones.

La bonne stabilité des émulsions a pu être obtenue grâce à l'utilisation de compositions selon l'invention contenant des polypentosides, choisis parmi les polyarabinosides et les polyxylosides, en une proportion bien déterminée.

En outre, la demanderesse a constaté que la stabilité des émulsions préparées à partir de la composition de l'invention, était peu sensible à la présence de sels. Ceci permet notamment de préparer des émulsions contenant des ingrédients chargés en sels avec lesquels il était jusqu'à présent parfois difficile d'obtenir des émulsions stables.

Par ailleurs, les compositions émulsionnantes de l'invention, constituées de polyglycosides et d'alcools gras, ne présentent pas les inconvénients des compositions à base de composés polyoxyéthylénés mentionnés précédemment. Elles ne sont pas irritantes, elles ne sont pas toxiques et elles sont biodégradables. En outre, les compositions émulsionnantes selon l'invention peuvent être utilisées comme compositions auto-émulsionnables.

La présente invention a donc pour objet une composition émulsionnante à base de polyglycosides, caractérisée en ce qu'elle comprend de 30 à 65 % en poids d'au moins un alcool gras de formule ROH, où R est un radical aliphatique, saturé ou insaturé ayant de 1 à 4 insaturations éthyléniques, linéaire ou ramifié, ayant de 12 à 22 atomes de carbone, le reste étant, sauf des impuretés, constitué :
- soit, d'un mélange de polyglycosides comprenant de 35 à 75 % en poids, par rapport aux polyglycosides, de polyhexosides de formule :

   R¹O(H)ₙ₁

   dans laquelle R¹ est un radical aliphatique, saturé ou insaturé ayant de 1 à 4 insaturations éthyléniques, linéaire ou ramifié, ayant de 12 à 22 atomes de carbone, H est le reste d'un hexose, ni est compris entre 1 et 5 ; et de 25 à 65 % en poids par rapport aux polyglycosides d'au moins un polypentoside de formule :

   R²O(P)ₙ₂

   dans laquelle R² est un radical aliphatique, saturé ou insaturé ayant de 1 à 4 insaturations éthyléniques, linéaire ou ramifié, ayant de 12 à 22 atomes de carbone, P est le reste d'un pentose choisi parmi l'arabinose et le xylose, n2 est compris entre 1 et 5 ;
- soit d'un mélange de polyglycosides de formule :

   R³O(G₁)ₐ(G₂)_{b}(G₃)_{c}(G₄)_{d}(G₅)ₑ

   dans laquelle R³ est un radical aliphatique, saturé ou insaturé ayant de 1 à 4 insaturations éthyléniques, linéaire ou ramifié, ayant de 12 à 22 atomes de carbone, G₁, G₂, G₃, G₄, G₅ sont indépendamment les uns des autres, des restes d'un ose choisi parmi les hexoses et les pentoses, ces derniers étant choisis parmi l'arabinose et le xylose, les hexoses représentant 35 à 75 % en poids de l'ensemble des restes d'oses G₁, G₂, G₃, G₄ et G₅ et les pentoses de 25 à 75 % en poids de l'ensemble des restes d'oses G₁, G₂, G₃, G₄ et G₅ ; a, b, c, d et e étant égaux à 0 ou à 1, la somme de a, b, c, d et e étant au moins égale à 1.

Chaque reste d'ose peut être sous forme isomérique α ou β, sous forme L OU D, et sous forme furanosique ou pyranosique. On préfère les hexoses de la série D, notamment le D-glucose, le D-galactose et le D-mannose. Pour les pentoses, on préfère le L-arabinose et le D-xylose présents abondamment dans les hémicelluloses de nombreux végétaux.

En raison de l'abondance du glucose sur le marché des sucres, de préférence, les glucosides représentent au moins 80 % des hexosides.

On préfère tout particulièrement les alcools gras de formule ROH ayant de 14 à 22 atomes de carbone et notamment les mélanges d'hexadécanol et d'octadécanol.

De plus, en raison de leur fabrication rapide, on préfère tout particulièrement les compositions de l'invention qui comportent des polyglycosides dont les radicaux R¹ et R² ou le radical R³ sont identiques au radical R de l'alcool gras.

La présente invention a pour premier objet une composition émulsionnante à base d'alcools gras et de polyglycosides. En raison de leur efficacité et de leur facilité de fabrication, On préfère tout particulièrement les compositions comprenant de 40 à 60 % en poids d'alcools gras par rapport au poids total de la composition et de préférence de 52 à 57 % d'alcool gras, le reste étant sauf des impuretés les polyglycosides.

Les compositions à base de polyglycosides et d'alcools gras de l'invention peuvent être préparés par simple mélange de leurs constituants en des proportions telles que mentionnées précédemment. Les techniques d'homogénéisation utilisées sont celles couramment utilisées pour mélanger des constituants solides ou liquides. Pour les constituants solides, on préfère cependant, si cela est possible, les mélanger à une température supérieure à leurs points de fusion sous forme liquide.

Cependant, à l'échelle industrielle, on prépare les compositions de l'invention selon l'une des deux voies classiquement utilisées pour la synthèse des polyglycosides d'alkyle.

La première voie consiste à mettre directement en contact le sucre réducteur et l'alcool gras en présence d'un catalyseur acide pour obtenir les polyglycosides.

La seconde voie consiste, dans un premier temps, à réaliser la glycosidation avec un alcool court qui répond à la formule R⁴OH, R⁴ étant un radical alkyle ayant de 1 à 5 atomes de carbone. Dans un second temps, on effectue une transglycosidation qui consiste à déplacer l'alcool court de formule R⁴OH par un alcool gras.

Chacune de ces deux voies peut être, le cas échéant, complétée par des opérations de neutralisation, de filtration, d'élimination de l'alcool gras en excès et de décoloration.

Avantageusement, notamment si on travaille à partir de sucres réducteurs cristallisés, on préfère utiliser la première voie directe qui présente l'avantage d'être plus rapide et facile à mettre en oeuvre. Cependant, lorsqu'on utilise des sucres réducteurs sous forme de sirops, on préfère utiliser la seconde voie qui permet d'obtenir un milieu réactionnel plus homogène et par conséquent des polyglycosides de meilleure qualité, ne contenant ou contenant très peu de produits de dégradation.

On entend par sucres réducteurs, les hexoses, les pentoses et les oligosaccharides correspondants présentant un hydroxyle anomère libre.

Lors de la glycosidation directe des sucres par l'alcool gras ou un mélange d'alcool gras, ou lors de la transglycosidation si le greffage est réalisé en deux étapes ; l'alcool gras est utilisé de préférence en excès (1 à 3 et de préférence 1,5 à 2 équivalents molaires par rapport aux sucres réducteurs), de telle sorte que le produit de la réaction contienne les quantités précédemment spécifiées d'alcool gras libre et de polyglycosides.

On pourrait également éliminer partiellement ou totalement l'alcool gras ou le mélange d'alcool gras en fin de synthèse, puis ajouter dans des proportions déterminées un alcool gras ou un mélange d'alcool gras différents ou identiques à ceux utilisés pendant la synthèse afin d'obtenir la composition de l'invention.

On préfère cependant la première solution qui consiste à utiliser l'alcool gras en excès de telle sorte que le produit de la réaction contienne les quantités spécifiées d'alcools gras et de polyglycosides.

En pratique, il existe trois principales voies pour obtenir les compositions de l'invention à partir de sucres réducteurs et d'alcools gras.

La première voie consiste à effectuer séparément la glycosidation des sucres réducteurs (hexoses tels que le glucose, le galactose, le mannose et les oligosaccharides correspondants, pentoses choisis parmi l'arabinose et le xylose et les oligosaccharides correspondants) par mise en contact avec un ou plusieurs alcools gras en présence d'un catalyseur acide habituellement utilisé pour les réactions de glycosylation. On préfère mettre l'alcool gras en excès (1 à 3 et de préférence 1,5 à 2 équivalents molaires par rapport aux sucres réducteurs) de telle sorte que le produit de la réaction contiennent les quantités spécifiées d'alcool gras libre et de polyglycosides. Après neutralisation du catalyseur acide, on obtient les polyhexosides de formule :

R¹O(H)ₙ₁

et les polypentosides de formule :

R²O(P)ₙ₂

Ensuite, 35 à 75 %, avantageusement 45 à 70 % et de préférence 50 à 65 % en poids par rapport au poids total des polyglycosides, de polyhexosides et 25 à 65 %, avantageusement 30 à 55 % et de préférence 35 à 50 % en poids par rapport au poids total des polyglycosides, de polypentosides sont mélangés en présence, si cela est nécessaire, d'alcool gras de formule ROH afin d'obtenir les compositions de l'invention.

La seconde voie consiste à mélanger 35 à 75 %, avantageusement 45 à 70 % et de préférence 50 à 65 % en poids par rapport au poids total de sucres réducteurs, d'hexoses et/ou des oligosaccharides correspondants, avec 25 à 65 %, avantageusement 30 à 55 % et de préférence 35 à 50 % en poids par rapport au poids total de sucres réducteurs, de pentoses et/ou des oligosaccharides correspondants et d'effectuer la glycosidation du mélange de sucres réducteurs ainsi obtenu. La glycosylation est réalisée en présence d'un catalyseur acide avec un excès (1 à 3 et de préférence 1,5 à 2 équivalents molaires par rapport aux sucres réducteurs) d'alcool gras de telle sorte que, de préférence, le produit de la réaction contiennent les quantités spécifiées d'alcools gras libres. La composition est neutralisée et si cela est nécessaire de l'alcool gras de formule ROH est ajouté afin d'obtenir les spécifications précitées.

Enfin , la troisième voie consiste à utiliser des sirops de mélanges de sucres réducteurs dérivés de matières premières végétales riches en amidon et en hémicellulose, ou de produits ou co-produits d'origine agricole tels que les produits ou co-produits du maïs (son, fibres et drèches de maïs), de l'orge (son), ou de co-produits du blé tels qu'ils sont décrits dans la demande de brevet EP O 699 472, contenant des hexoses et des pentoses et d'effectuer la glycosidation de ces sirops de sucres réducteurs avec des alcools gras afin d'obtenir les compositions de l'invention.

On entend par produits du maïs, la totalité de la plante et/ou les organes constitutifs (rafles, spathes, tiges, feuilles) qui peuvent être récoltés directement ou provenir de déchets après séparation des grains.

On peut également utiliser de la fibre ou du son de maïs. On entend par fibre de maïs les composés obtenus au cours d'un procédé de fractionnement, visant notamment à produire de l'amidon.

Par drèches de maïs, on entend les co-produits obtenus soit par fermentation alcoolique, soit après obtention d'amidon par voie humide dans une amidonnerie et qui sont notamment constitués de mélanges d'hémicellulose et d'amidon.

Selon une caractéristique particulière de l'invention, on préfère cette troisième voie qui présente l'avantage d'utiliser des sirops de sucres réducteurs (constitués d'hexoses et de pentoses) moins chers que les hexoses (glucose) et les pentoses du marché et de permettre ainsi d'obtenir des compositions à base de polyglycosides bon marché.

On préfère en particulier utiliser des sirops de sucres réducteurs obtenus par hydrolyse de co-produits d'origine végétale contenant notamment de l'amidon et de l'hémicellulose.

On préfère tout particulièrement utiliser des sirops de sucres réducteurs dérivés du blé, notamment du son de blé, de fibres de blé tels qu'elles sont définies dans la demande EP 0699 472, des sirops de sucres dérivés des sous-produits du maïs (son de maïs, drèches de maïs).

Selon une caractéristique avantageuse, liée notamment à l'origine naturelle de la composition des sirops de sucres utilisés pour la préparation des polyglycosides des compositions de l'invention, on préfère tout particulièrement les compositions à base de polyglycosides comprenant de 45 à 70 % en poids de polyhexosides par rapport aux polyglycosides et de 30 à 55 % en poids de polypentosides et notamment de 50 à 65 % en poids de polyhexosides et de 45 à 50 % en poids de polypentosides.

C'est pourquoi, compte tenu des remarques précédentes, selon une caractéristiques particulière de l'invention, les compositions de l'invention sont préférentiellement obtenues par la mise en contact d'un sirop de sucres réducteurs dérivé de matière première végétale contenant de l'amidon et de l'hémicellulose de telle sorte que les polyglycosides des compositions de l'invention contiennent les quantités spécifiques de polyhexosides et de polypentosides, avec un alcool court de formule R⁴OH, en présence de 0,1 à 5 % en poids par rapport à la matière sèche des sucres, d'un catalyseur acide tel que l'acide sulfurique, l'acide chlorhydrique, un acide sulfonique tel que l'acide méthanesulfonique, l'acide hypophosporeux ou de tout autre catalyseur acide permettant d'effectuer une glycosidation et leurs mélanges, à une température comprise entre 50 et 110°C.

On réalise par la suite une transglycosidation, sous pression réduite (2 à 300 mb), à une température comprise entre 50 et 100°C, par un alcool gras ou un mélange d'alcools gras de formule ROH mis en excès (1 à 3 et de préférence 1,5 à 2 équivalents molaires par rapport aux sucres réducteurs) de telle sorte que le produit de la réaction contienne les quantités précédemment spécifiées d'alcool gras et de polyglycosides.

Le catalyseur acide est ensuite neutralisé. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude, ou une base organique telle que la triéthanolamine. On peut par la suite, si cela est nécessaire, évaporer une partie ou la totalité de l'alcool gras libre et décolorer la composition en présence de peroxyde d'hydrogène par exemple.

La composition selon l'invention se présente en fin de synthèse, selon l'alcool gras ou le mélange d'alcools gras utilisé, sous la forme d'une cire solide, pâteuse ou liquide. A partir d'une cire solide, il est possible, notamment pour une utilisation ultérieure plus aisée, d'obtenir de la poudre, des paillettes ou encore des perles. On préfère isoler la composition sous forme de poudre très facile à utiliser par la suite, notamment pour la fabrication d'émulsions.

Selon un autre aspect de l'invention, les compositions à base de polyglycosides de l'invention peuvent être utilisées pour la préparation d'émulsions comprenant au moins une phase aqueuse et une phase huileuse caractérisées par le fait qu'elle contiennent :
- de 2 à 60 % en poids d'au moins une huile
- de 1 à 10 % en poids de la composition à base de glycosides d'alkyle de l'émulsion
- éventuellement d'autres compositions émulsionnantes telles que des tensioactifs polyoxyéthylénés, des mélanges d'alcools gras et de tensioactifs polyoxyéthylénés et glycosidiques, des cires...
Le reste étant essentiellement constitué par une phase aqueuse.

La phase huile de l'émulsion peut être constituée par une huile choisie parmi :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de noisette, l'huile de palme, le beurre de karité, l'huile de noyau d'abricot, l'huile de calophyllum, l'huile de carthame, l'huile d'avocat, des dérivés de ces huiles comme les huiles hydrogénées,
- les huiles végétales riches en acide linoléique, telles que l'huile de noix, l'huile de pépins de cassis, l'huile de germes de blé, l'huile de tournesol, l'huile de germes de maïs, l'huile de soja, l'huile de coton, l'huile de luzerne, l'huile d'orge, l'huile de pépins de raisin, l'huile de pavot, l'huile de potimarron, l'huile de sézame, l'huile de seigle, l'huile d'onagre, l'huile de carthane, l'huile de passiflore, des dérivés de ces huiles comme les huilés hydrogénées,
- les huiles d'origine animale (suif, huiles de poisson...),
- les huiles minérales, telles que l'huile de paraffine, l'huile de vaseline et les huiles minérales notamment issues de coupes pétrolières.
- les huiles synthétiques, comme les poly-α-oléfines,
- les dérivés de la lanoline,
- les diols comme le 1,2-propanediol, le 1,3-butanediol,
- les alcools tels que l'alcool cétylique, l'alcool stéarylique, l'alcool oléique,
- les polyéthylène glycols ou polypropylène glycols,
- les esters gras tels que les myristates alkyle notamment le myristate de butyle, le myristate de propyle, les palmitates d'alkyle comme le palmitate d'isopropyle, les stéarates d'alkyle notamment le stéarate d'hexadécyle, les oléates d'alkyle, notamment l'oléate de dodécyle, les laurates d'alkyle, notamment le laurate d'hexyle, le dicaprylate de propylène glycol, le cocoate d'éthyl-2-hexyle, les esters de l'acide lactique, de l'acide béhennique, de l'acide isostéarique tel que l'isostéarate d'isostéaryle,
- les huiles silicones regroupant les polydiméthylsiloxanes cycliques, les polydiméthylsiloxanes α-ω hydroxylées, les polydiméthylsiloxanes α-ω triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POULENC ou DC 190® commercialisée par DOW CORNING) ou les dérivés mixtes de silicones comme les copolymères mixtes polyalkylméthylsiloxanes-silicones copolyéthers.

Bien entendu, les émulsions préparées à partir de la composition de l'invention, peuvent également contenir un ou plusieurs adjuvants cosmétiques, lipophiles ou hydrophiles classiques, notamment ceux qui sont déjà utilisés de manière habituelle dans la fabrication et l'obtention d'émulsions. Parmi les adjuvants cosmétiques classiques susceptibles d'être contenus dans la phase aqueuse et/ou la phase grasse des émulsions, on peut citer notamment :
- les épaississants et les gélifiants ioniques, ou non ioniques, comme les dérivés de cellulose (carboxyméthylcellulose, hydroxyéthylcellulose), de guar (hydroxypropylguar, carboxyméthylguar, carboxyméthylhydroxypropylguar...), de caroube, les exsudats d'arbres (gomme arabique, le karaya...), les extraits d'algues marines (alginates, carraghénates...), les exsudats de micro-organismes (gomme de xanthane),
- les agents hydrotropes, comme les alcools courts en C2-C8, en particulier l'éthanol, les diols et glycols comme le diéthylène glycol, dipropylèneglycol,...
- des agents hydratants ou humectants pour la peau comme le glycérol, le sorbitol, le collagène, la gélatine, l'aloe vera, l'acide hyaluronique, l'urée ou des agents protecteurs de la peau, comme les protéines ou hydrolysats de protéines, les polymères cationiques, comme les dérivés cationiques du guar (JAGUAR C13S®, JAGUAR C162®, HICARE 1000® commercialisés par RHONE-POULENC),
- des glycolipides tels que les sophoroses lipides,
- les poudres ou des particules minérales comme du carbonate de calcium, les oxydes minéraux sous forme de poudre ou sous forme colloïdale (particules de taille inférieure ou de l'ordre de un micromètre, parfois de quelques dizaines de nanomètres) comme du dioxyde de titane, de la silice, des sels d'aluminium utilisés généralement comme antitranspirants, du kaolin, du talc, les argiles et leurs dérivés,...
- les agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques sont généralement introduits dans ces compositions à hauteur de 0,01 à 3% en poids.
Au lieu de ces agents chimiques, on peut parfois utiliser des agents modifiant l'activité de l'eau et augmentant fortement la pression osmotique comme les carbohydrates ou des sels.
- les filtres solaires organiques actifs dans l'UV-A et/ou l'UV-B pour protéger la peau ou les cheveux des agressions du soleil et des rayons UV, comme les composés autorisés dans la directive Européenne N° 76/768/CEE, ses annexes et les modifications ultérieures de cette directive,
- les monopigments minéraux photoprotecteurs, comme le dioxyde de titane ou les oxydes de cérium sous forme de poudre ou de particules colloïdales.
- les adoucissants, les antioxydants, les agents autobronzant comme la DHA, les agents répulsifs contre les insectes, les vitamines, les parfums, les charges, les séquestrants, les colorants, les agents tampon...
- les abrasifs tels que les noyaux d'abricots broyés, les microbilles...

Selon une utilisation préférée de l'invention, le pourcentage en poids de la composition émulsionnante à base de polyglycosides représente de 2 à 6 % en poids et de préférence de 3 à 4 % du poids total de l'émulsion.

Selon une autre utilisation préférée de la composition de l'invention, la proportion en huile est comprise entre 10 et 40 % en poids par rapport au poids total de l'émulsion.

Trois principales méthodes de fabrication des émulsions sont proposées :
- La première consiste à chauffer tous les ingrédients en même temps à une température comprise entre 50 et 90°C, puis à homogénéiser avec un agitateur rotatif à pâle tournant de 500 à 15000 rpm, en particulier de 1000 à 2000 rpm, à une température comprise entre 50 et 90°C et enfin à refroidir sous agitation lente ( de 100 à 1000, en particulier de 300 à 500 rpm) jusqu'à une température de l'ordre de 25°C. Dans le cas où l'homogénéisation est vive à chaud, il n'est pas toujours utile d'agiter l'émulsion pendant son refroidissement.
- La seconde consiste à travailler par inversion de phase. Dans ce cas, les phases lipophile et hydrophile sont chauffées séparément à une température comprise entre 50 et 90°C. La phase lipophile, qui contient la composition de l'invention, est mise sous agitation vive avec un agitateur rotatif à pâle tournant de 500 à 15000 rpm, en particulier de 1000 à 2000 rpm et on ajoute lentement, à un débit tel que la phase hydrophile est instantanément absorbée par la phase lipophile, à cette phase la phase hydrophile jusqu'à l'inversion de phase caractérisée par un changement brutal de la viscosité. L'addition peut être ensuite plus rapide à un débit tel que la phase hydrophile stagne 1 à 3 secondes au dessus de la phase lipophile si l'ajout a lieu par le dessus. On laisse enfin refroidir l'émulsion sous agitation lente ( de 100 à 1000, en particulier de 300 à 500 rpm) jusqu'à une température de l'ordre de 25°C.
- La troisième méthode 'est réalisée par dispersion. Dans ce cas les phases lipophile et hydrophile (qui contient la composition émulsionnante de l'invention), sont chauffées séparément à une température comprise entre 50 et 90°C. La phase hydrophile est mise sous agitation avec un agitateur rotatif à pâle tournant de 500 à 15000 rpm, en particulier de 1000 à 2000 rpm et on y ajoute progressivement, à un débit tel que la phase lipophile est instantanément absorbée par la phase hydrophile, la phase lipophile. On laisse ensuite refroidir l'émulsion sous agitation lente ( de 100 à 1000, en particulier de 300 à 500 rpm) jusqu'à une température de l'ordre de 25°C.

Selon un autre aspect de l'invention, la composition à base de polyglycosides peut être utilisée comme base auto-émulsionnable pour la préparation d'émulsions par dispersion à chaud des compositions de l'invention, par exemple entre environ 50 et 90 °C, dans de l'eau ou un milieu polaire approprié, par simple agitation, notamment mécanique ou par sonication. Si on disperse la composition dans l'eau par agitation ou par sonication à une température proche du point de fusion de la composition émulsionnante, on obtient des dispersions riches en vésicules.

Les émulsions préparées à partir de la composition de l'invention peuvent être utilisées dans différentes applications cosmétiques ou dermatologiques, par exemple sous forme de crèmes pour le visage, pour le corps, pour le cuir chevelu ou pour la chevelure ou sous forme de lait pour le corps ou pour le démaquillage ou encore sous forme de pommades par exemple à usage pharmaceutique. Ces émulsions peuvent également être utilisées pour le maquillage, notamment sous forme de fonds de teint, après addition de pigments. Elles peuvent aussi être utilisées comme crèmes solaires après addition de filtres UVA et/ou UVB et/ou DHA, ou comme crèmes ou laits après soleil après addition de composés apaisants tels que le panthénol ou le beurre de Karité.

Les émulsions peuvent également contenir des agents tensioactifs lavants, moussants ou détergents ioniques ou non ioniques tels que le lauryléther sulfate de sodium, les alkyl-bétaïnes, les APG... pour faire des émulsions lavantes telles que les crèmes lavantes hydratantes, ou des émulsions pour le rasage.

Les émulsions peuvent contenir en outre, en vue d'augmenter leurs qualités cosmétiques, une cire cosmétique telle que par exemple de la cire de riz, de la cire de candellila, de la cire du Japon. La proportion de cire est généralement comprise entre 0,5 et 3%, et de préférence entre 1 et 2% en poids par rapport au poids total de l'émulsion.

Les compositions à base de polyglycosides et d'alcools gras de l'invention peuvent être utilisés également dans des formulations où il est nécessaire de maintenir en suspension dans l'eau des solides finement divisés, comme les formulations de matières actives agrochimiques (herbicides, insecticides, fongicides,...) connues sous le nom générique de "suspensions concentrées". Outre un tensioactif dispersant, on retrouve comme additifs dans une formulation de suspension concentrée, des additifs comme ceux décrits dans la brochure commerciale "Auxiliaries for agrochemical formulations" éditées par RHONE-POULENC GERONAZZO SpA. On peut citer par exemple un tensioactif mouillant, pris parmi les dérivés alcoylés d'alcools arylaliphatiques, les dérivés aryl sulfonés comme l'isopropylnaphtalène sulfonate de sodium, commercialisé sous le nom SUPRAGIL WP® par RHONE-POULENC GERONAZZO, les dialkyl sulfosuccinates comme le di-éthyl-2-hexyl sulfosuccinate de sodium, des polymères dispersants, comme les acides polyacryliques et leurs sels, les copolymères anhydride (ou acide) maléique - diisobutylène et leurs sels comme le GEROPON T36® (RHONE-POULENC GERONAZZO), les méthylnaphtalène sulfonates de sodium condensés comme le SUPRAGIL MNS90® (RHONE-POULENC GERONAZZO), les polymères dispersants dérivés de la lignine comme les lignosulfonates de sodium ou de calcium ou d'autres tensioactifs dispersants comme les dérivés alcoxylés, éventuellement sulfatés ou phosphatés de tristyrylphénols. On peut trouver en outre dans ces formulations, des additifs antigels comme le propylène glycol et des additifs épaississants, modifiant le comportement rhéologique de la suspension comme la gomme xanthane, les dérivés de la cellulose (carboxyméthylcellulose), la gomme guar ou ses dérivés, des argiles ou des argiles modifiées comme la bentonite et les bentones.
Parmi les matières actives pouvant être formulées ainsi, on trouve généralement celles dont le point de fusion est supérieur à 45°C préférentiellement supérieur à 60°C et dont la solubilité dans l'eau est inférieure à 10 g/l, préférentiellement inférieure à 1 g/l. Les matières actives phytosanitaires concernées sont les herbicides, les fongicides et insecticides, tels que ceux décrits dans THE PESTICIDE MANUAL (9° édition, C.R. WORKLING et R.J. HANCE, éditeurs, publié par The British Crop Protection Council) et répondant aux critères ci-dessus.

Les exemples suivants ont pour but d'illustrer la présente invention.

### Exemple 1 de synthèse

### Procédé de préparation de polyglucosides de cétéaryle

85,3 g de D-glucose anhydre sont mis en suspension dans 94,6 g de *n*-butanol en présence de 1,7 g d'acide sulfurique. Le milieu réactionnel est porté à reflux du butanol jusqu'à ce que le milieu réactionnel devienne limpide. Celui-ci est ensuite ajouté à 90°C, en 90 minutes et sous pression réduite (50 mb), à 225 g d'alcool gras (hexadécanol : 70 %, octadécanol : 30 %) contenant 0,85 g d'acide sulfurique et le butanol est éliminé sous pression réduite. Le milieu réactionnel est maintenu dans les mêmes conditions pendant 30 minutes après la fin de l'addition. L'acidité du milieu est neutralisée par une solution aqueuse de soude à 30,5 % (3,9 g) jusqu'à pH 7 à 8. Le produit de la réaction appelé composition à base de glucosides de cétéaryle (310 g) se présente sous la forme d'une pâte solide qui contient 52 % en poids d'alcool gras résiduel. Celui-ci est ramené ensuite à 55 % par rapport à l'ensemble du produit de la réaction par addition de 9,3 g d'un mélange d'hexadécanol et d'octadécanol (70/30) et est broyé afin d'obtenir une poudre de granulométrie inférieure à 800 µm.

### Exemple 2 de synthèse

### Procédé de préparation de polyxylosides de cétéaryle

96,6 g de D-xylose anhydre sont mis en suspension dans 128,7 g de *n*-butanol en présence de 1,93 g d'acide sulfurique. Le milieu réactionnel est porté à reflux du butanol jusqu'à ce que le milieu réactionnel devienne limpide. Celui-ci est ensuite ajouté à 80°C, en 90 minutes et sous pression réduite (50 mb), à 306 g d'alcool gras (hexadécanol : 70 %, octadécanol : 30 %) contenant 0,97 g d'acide sulfurique et le butanol est éliminé sous pression réduite. Le milieu réactionnel est maintenu dans les mêmes conditions pendant 30 minutes après la fin de l'addition. L'acidité du milieu est neutralisée par une solution aqueuse de soude à 30,5 % (4,4 g) jusqu'à pH 7 à 8. Le produit de la réaction appelé composition à base de xylosides de cétéaryle (401 g) se présente sous la forme d'une pâte solide qui contient 46,5 % en poids d'alcool gras résiduel. Celui-ci est ramené ensuite à 55 % par rapport à l'ensemble du produit de la réaction par addition de 34 g d'un mélange d'hexadécanol et d'octadécanol (70/30) et est broyé afin d'obtenir une poudre de granulométrie inférieure à 800 µm.

### Exemple 3 de synthèse

### Procédé de préparation de polyarabinosides de cétéaryle

90,7 g de L-arabinose anhydre sont mis en suspension dans 120,8 g de *n*-butanol en présence de 1,81 g d'acide sulfurique. Le milieu réactionnel est porté à reflux du butanol jusqu'à ce que le milieu réactionnel devienne limpide. Celui-ci est ensuite ajouté à 80°C, en 90 minutes et sous pression réduite (50 mb), à 287,2 g d'alcool gras (hexadécanol : 70 %, octadécanol : 30 %) contenant 0,91 g d'acide sulfurique et le butanol est éliminé sous pression réduite. Le milieu réactionnel est maintenu dans les mêmes conditions pendant 30 minutes après la fin de l'addition. L'acidité du milieu est neutralisée par une solution aqueuse de soude à 30,5 % (4,2 g) jusqu'à pH 7 à 8. Le produit de la réaction appelé composition à base d'arabinosides de cétéaryle (376 g) se présente sous la forme d'une pâte solide qui contient 50,4 % en poids d'alcool gras résiduel. Celui-ci est ramené ensuite à 55 % par rapport à l'ensemble du produit de la réaction par addition de 17,3 g d'un mélange d'hexadécanol et d'octadécanol (70/30) et est broyé afin d'obtenir une poudre de granulométrie inférieure à 800 µm.

### Exemple 1

### Procédé de préparation d'une composition à base de polyglycoside et d'alcool cétéarylique selon l'invention

55 g de la composition à base de polyglucoside de cétéaryle de l'exemple 1 de synthèse, 30 g de la composition à base de polyxylosides de cétéaryle de l'exemple 2 de synthèse et 15 g de la composition à base de polyarabinosides de cétéaryle de l'exemple 3 de synthèse, sont mélangés afin d'obtenir 100 g de la composition à base de polyglycosides caractérisée en ce qu'elle comprend de 55 % en poids d'alcool gras, le reste étant constitué de polyglycosides de cétéaryle comprenant 55 % en poids, par rapport aux polyglycosides, de polyhexosides de cétéaryle et 45 % en poids de polypentosides de cétéaryle (30 % en poids de polyxylosides de cétéaryle et 15 % en poids de polyarabinosides de cétéaryle).

### Exemple 2

### Procédé de préparation d'une composition à base de polyglycoside de cétéaryle et d'alcool cétéarylique selon l'invention

A une suspension d'un mélange de 65 g de D-glucose anhydre, 5 g de D-galactose, 20 g de D-xylose et 10 g de L-arabinose dans 284,5 g d'un mélange d'hexadécanol/octadécanol (50/50 poids/poids), on ajoute 3 g d'acide sulfurique à 95%. Le milieu réactionnel est porté à 90°C sous pression réduite (30 mbars) pendant 2h30mn. Ensuite, à la même température, le catalyseur acide est neutralisé par 6,1 g de soude aqueuse à 30,5 % et le produit décoloré en présence de peroxyde d'hydrogène et de soude. Après refroidissement à 20°C et broyage du solide obtenu, on recueille 380 g de la composition qui contient 54 % en poids par rapport au poids total de la composition d'alcool gras.

### Exemple 3

### Procédé de préparation d'une composition à base de polyglycosides dérivés du son de blé selon l'invention

On prépare une suspension de son de blé à 20 % de matière sèche (100 kg de matière sèche son pour 500 kg de suspension) dans une solution d'acide sulfurique (10 % H₂SO₄ / MS son). Le milieu réactionnel est homogénéisé dans un réacteur agité, puis porté à une température de 130 °C pendant 30 minutes. Après l'hydrolyse, le mélange est chaulé jusqu'à pH 5 avec un lait de chaux et pressé sur un filtre presse pour séparer les gâteaux du jus 1. Ce jus 1 est ensuite déminéralisé par échange d'ions successivement sur une résine cationique forte (A 200), une résine anionique faible (A368S) et enfin une résine cationique forte (A 200). Le jus 2 obtenu est ensuite concentré jusqu'à 74,9 % de matière sèche par évaporation de l'eau sous pression réduite et à 50°C. La composition du sirop 3 obtenu est reportée dans le tableau suivant :

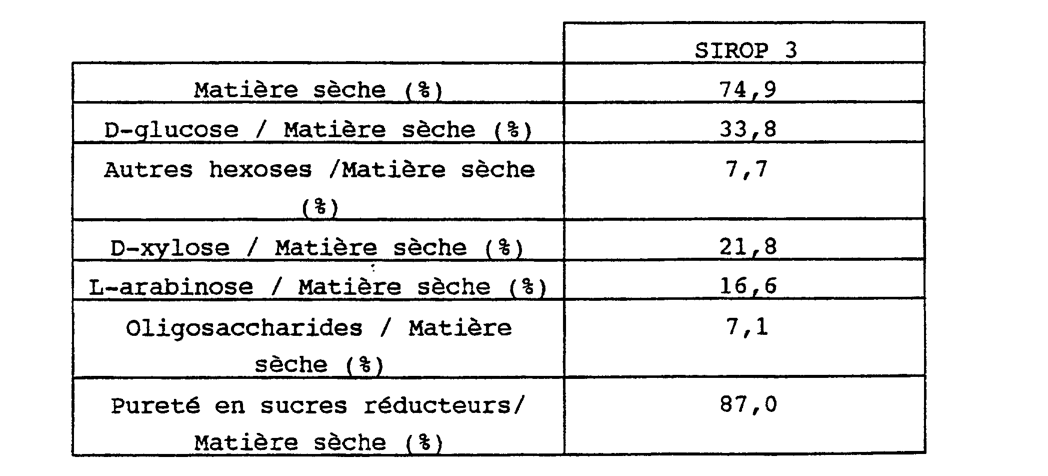

350 g de ce sirop 3 sont ensuite ajoutés goutte à goutte en 1 heure 30 à 338,3 g de *n*-butanol contenant 5,2 g d'acide sulfurique et 22 g d'eau à une température de l'ordre de 100 à 105°C. L'eau est éliminée au cours de la réaction par distillation azéotropique du mélange eau/butanol. On ajoute ensuite le milieu réactionnel obtenu à 767,3 g d'alcool gras (hexadécanol : 30 %, octadécanol : 70 % en poids) contenant 2,6 g d'acide sulfurique, à une température de 90°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C et broyage du solide obtenu, on recueille 1020 g de la composition qui contient 57 % en poids par rapport au poids total de la composition d'alcool gras.

### Exemple 4

### Procédé de préparation d'une composition à base de polyglycosides dérivés du son de blé selon l'invention

100 g du sirop 3 de l'exemple 3 sont ajoutés goutte à goutte en 1 heure 30 à 96,7 g de *n*-butanol contenant 1,5 g d'acide sulfurique et 6,3 g d'eau à une température de l'ordre de 100 à 105°C. L'eau est éliminée au cours de la réaction par distillation azéotropique du mélange eau/butanol. On ajoute ensuite le milieu réactionnel obtenu à 165,3 g d'alcool gras (dodécanol : 27 %, tétradécanol : 23 %, hexadécanol : 26 %, octadécanol : 24 % en poids) à une température de 90°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8. Les alcools gras présents dans le mélange réactionnel sont partiellement éliminés par distillation sur un évaporateur couche mince. On obtient un mélange contenant 34 % en poids d'alcools gras dont :
Dodécanol : 0,3 g
Tétradécanol : 2,6 g
Hexadécanol : 27,9 g
Octadécanol : 32,7 g
On ajoute au milieu 56,5 g d'un mélange d'hexadécanol/octadécanol (50/50, poids/poids) en vue d'obtenir 243 g de la composition suivante contenant :
0,1 % en poids de dodécanol libre
1,1 % en poids de tétradécanol libre
23,0 % en poids d'hexadécanol libre
25,1 % en poids d'octadécanol libre
Le reste étant sauf des impuretés des polyglycosides.

### Exemple 5

### Procédé de préparation d'une composition à base de polyglycosides dérivés de fibres de blé selon l'invention

On prépare une suspension de fibres de blé (telles que définies dans la demande de brevet EP 0 699 472) à 20 % de matière sèche (100 kg de matière sèche de fibres pour 500 kg de suspension) dans une solution d'acide sulfurique (10 % H₂SO₄/MS des fibres). Le milieu réactionnel est homogénéisé dans un réacteur agité, puis porté à une température de 130 °C pendant 30 minutes. Après l'hydrolyse, le mélange est chaulé jusqu'à pH 5 avec un lait de chaux et pressé sur un filtre presse pour séparer les gâteaux du jus 1. Ce jus 1 est ensuite déminéralisé par échange d'ions sur une résine cationique forte (A 200), et décoloré en présence de 1 % en poids par rapport au poids total de jus de charbon actif (CG1 NORIT) pendant 30 minutes à 20°C. Le charbon est ensuite éliminé par passage sur un filtre presse puis sur un filtre à plaques (seuil de coupure : 0,2 µm) pour obtenir un jus 2. Ce jus 2 est ensuite concentré jusqu'à 74,1 % de matière sèche par évaporation de l'eau sous pression réduite à 50°C. La composition du sirop 3 obtenu est reportée dans le tableau suivant :

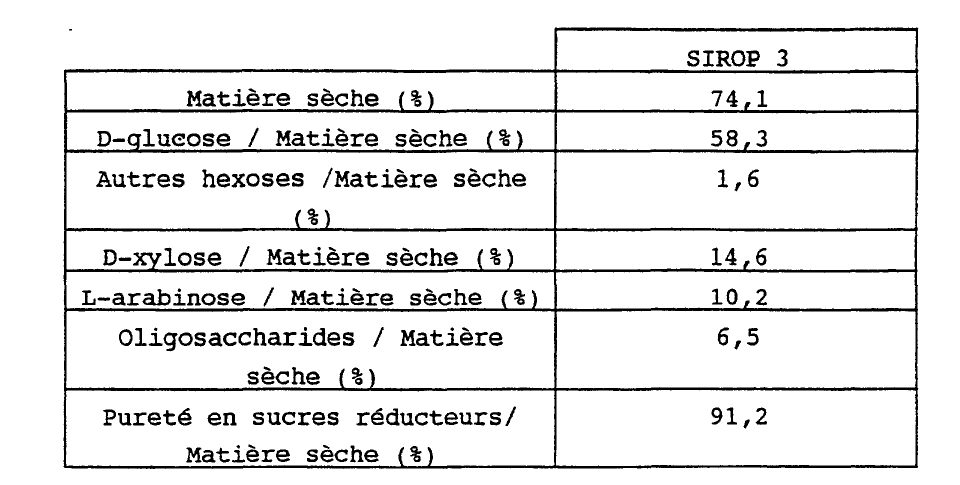

150 g de ce sirop 3 sont ensuite ajoutés goutte à goutte en 1 heure 30 à 138,7 g de *n*-butanol contenant 1,3 g d'acide sulfurique et 9 g d'eau à une température de l'ordre de 100 à 105°C. L'eau est éliminée au cours de la réaction par distillation azéotropique du mélange eau/butanol. On ajoute ensuite le milieu réactionnel obtenu à 308,4 g d'alcool gras (hexadécanol: 70 %, octadécanol : 30 % en poids) contenant 1,1 g d'acide sulfurique, à une température de 90°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C et broyage du solide obtenu, on recueille 420 g de la composition qui contient 56 % en poids par rapport au poids total de la composition d'alcools gras libres.

### Exemple 6

### Procédé de préparation d'une composition à base de polyglycoside dérivés de fibres de blé selon l'invention

100 g du sirop 3 de l'exemple 5 sont ajoutés goutte à goutte en 1 heure 30 à 92,5 g de *n*-butanol contenant 0,9 g d'acide sulfurique et 6 g d'eau à une température de l'ordre de 100 à 105°C. L'eau est éliminée au cours de la réaction par distillation azéotropique du mélange eau/butanol. On ajoute ensuite le milieu réactionnel obtenu à 214,9 g d'alcool oléique contenant 0,7 g d'acide sulfurique, à une température de 90°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C et broyage du solide obtenu, on recueille 290 g de la composition qui contient 55,5 % en poids par rapport au poids total de la composition d'alcool gras libre.

### Exemple 7

### Procédé de préparation d'une composition à base de polyglycoside dérivés de fibres de blé selon l'invention

150 g du sirop 3 de l'exemple 5 sont ajoutés goutte à goutte en 1 heure 30 à 138,7 g de *n*-butanol contenant 1,3 g d'acide sulfurique et 9 g d'eau à une température de l'ordre de 100 à 105°C. L'eau est éliminée au cours de la réaction par distillation azéotropique du mélange eau/butanol. On ajoute ensuite le milieu réactionnel obtenu à 357,3 g d'alcool gras (hexadécanol : 50 %, octadécanol : 50 % % en poids) contenant 1,1 g d'acide sulfurique, à une température de 90°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C, on recueille 470 g de la composition qui contient 60 % en poids par rapport au poids total de la composition d'alcool gras libre.

### Exemple 8

### Procédé de préparation d'une composition à base de polyglycoside dérivés de son de maïs selon l'invention

On met en contact 2 kg de son de maïs séché à 92 % de matière sèche contenant par rapport à la matière sèche 30 % d'amidon, 8,5 % de protéines, 36 % d'hémicellulose et 10 kg de solution d'acide sulfurique à 2 %. Le milieu réactionnel est homogénéisé dans un réacteur agité, puis porté à une température de 140 °C pendant 30 minutes. Après l'hydrolyse, le mélange est pressé sur une presse de laboratoire MARREL pour séparer le marc 1 du jus 1. On recueille un jus et un marc à respectivement 19,5 % et 45 % de matière sèche.
Le jus 1 est ensuite déminéralisé par échange d'ions sur une résine cationique forte (A 200), une résine anionique faible (A 368S) et enfin sur une résine cationique forte (A 200). Le jus 2 obtenu est ensuite concentré jusqu'à 73,3 % de matière sèche par évaporation de l'eau sous pression réduite et à 50°C. La composition du sirop 3 obtenu est reportée dans le tableau suivant :

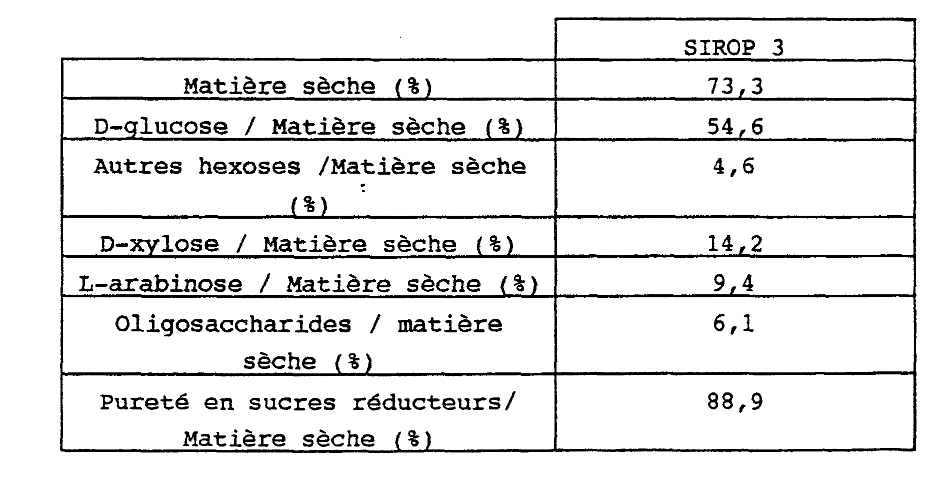

150 g de ce sirop 3 sont ensuite ajoutés goutte à goutte en 1 heure 30 à 138,5 g de *n*-butanol contenant 2,2 g d'acide sulfurique et 9 g d'eau à une température de l'ordre de 100 à 105°C. L'eau est éliminée au cours de la réaction par distillation azéotropique du mélange eau/butanol. On ajoute ensuite le milieu réactionnel obtenu à 306,7 g d'alcool gras (tétradécanol : 10 %, hexadécanol : 55 %, octadécanol : 35 % en poids) contenant 1,1 g d'acide sulfurique, à une température de 90°C et en 2 heures. Le butanol est éliminé sous pression réduite en continu pendant l'addition. Ensuite, à la même température, l'acidité du milieu est neutralisée par une solution aqueuse de soude à 30,5 % % jusqu'à pH 7 à 8 et le produit est décolorée en présence d'eau oxygénée. Après refroidissement à 20°C et broyage du solide obtenu, on recueille 421 g de la composition qui contient 56 % en poids par rapport au poids total de la composition d'alcool gras.

### Exemple 9

### Procédé de préparation d'une composition à base de polyglycoside dérivés de drèches de maïs selon l'invention

On prépare une suspension de drèches de maïs (2 kg de matière sèche de drèches de maïs pour 10 kg de suspension) dans une solution d'acide sulfurique (10 % H₂SO₄ / MS de drèches). Le milieu réactionnel est homogénéisé dans un réacteur agité, puis porté à une température de 120 °C pendant 1 heure. Après l'hydrolyse,le milieu réactionnel est chaulé jusqu'à pH 4,3 à l'aide d'un lait de chaux à 18 % de matière sèche et est pressé sur une presse de laboratoire MARREL pour séparer le marc 1 du jus 1
Le jus 1 est ensuite déminéralisé par échange d'ions sur une résine cationique forte (A 200), puis par électrodialyse. Le jus 2 obtenu est ensuite concentré jusqu'à 77,1 % de matière sèche par évaporation de l'eau sous pression réduite et à 50°C. La composition du sirop 3 obtenu est reportée dans le tableau suivant :

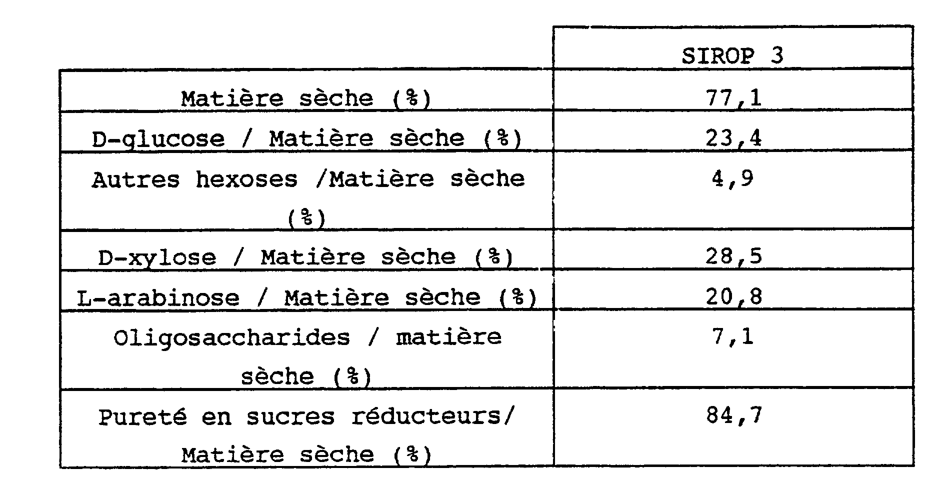

100 g de ce sirop 3 sont ensuite ajoutés goutte à goutte en 1 heure à 102,4 g de *n*-butanol contenant 1,5 g d'acide sulfurique et 6,6 g d'eau à une température de l'ordre de 100 à 105°C. L'eau est éliminée au cours de la réaction par distillation azéotropique du mélange eau/butanol. On ajoute ensuite le milieu réactionnel obtenu à 227,7 g d'alcool gras (hexadécanol : 70 %, octadécanol : 30 % en poids) à une température de 80°C et en 2 heures. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C et broyage du solide obtenu, on recueille 305 g de la composition qui contient 58 % en poids par rapport au poids total de la composition d'alcool gras.

### Exemple 10

### Exemple d'utilisation de la composition auto-émulsionnable de l'exemple 9 pour la préparation d'une crème

2 g de la composition autoémulsionnable de l'exemple 9 sont mis en suspension dans 48 g d'eau osmosée. Le mélange est porté à 50 °C et est ensuite agité (500 rpm) pendant 2 minutes. L'émulsion ainsi formée est ensuite refroidie à température ambiante. Sa viscosité, après 1 jour de mûrissement, est de 10300 cp (BROOKFIELD DV II - 25°C - 12 rpm - module 3).

### Exemple 11

### Exemple d'utilisation de la composition auto-émulsionnable de l'exemple 9 pour la préparation d'un lait fluide

1 g de la composition autoémulsionnable de l'exemple 9 sont mis en suspension dans 49 g d'eau osmosée. Le mélange est porté à 50 °C et est ensuite agité (500 rpm) pendant 2 minutes. L'émulsion ainsi formée ets ensuite refroidie à température ambiante. Sa viscosité, après 1 jour de mûrissement, est de 3000 cp (BROOKFIELD DV II - 25°C - 12 rpm - module 3).

### Exemple 12

### Exemple d'utilisation de la composition de l'exemple 3 pour la préparation d'une crème

Chauffer 2 g de la composition à base de polyglycosides de l'exemple 3, 7,5 g d'isostéarate d'isostéaryle et 40,5 g d'eau osmosée en même temps à une température de 70°C, puis à homogénéiser (1500 rpm) à la même température pendant 1 minute et enfin à refroidir sous agitation lente (300 rpm) jusqu'à une température de l'ordre de 25°C. Après 1 jour de mûrissement, la viscosité de l'émulsion ainsi formée est de 9500 cp (BROOKFIELD DV II - 25°C - 12 rpm - module 3).

### Exemple 13

### Exemple d'utilisation de la composition de l'exemple 3 pour la préparation d'une crème

La phase lipophile (15 g d'isostéarate d'isostéaryle) qui contient 4 g de la composition à base de polyglycosides de l'exemple 3 et la phase hydrophile (81 g d'eau osmosée) sont chauffées séparément à une température de 70°C. La phase lipophile est mise sous agitation vive (1500 rpm) et on y ajoute en 2 minutes la phase hydrophile jusqu'à l'inversion de phase caractérisée par un changement brutal de la viscosité. L'addition peut être ensuite plus rapide (1 minute). On laisse enfin refroidir l'émulsion sous agitation lente (300 rpm) jusqu'à une température de l'ordre de 25°C.
Après 1 jour de mûrissement, la viscosité de l'émulsion ainsi formée est de 12000 cp (BROOKFIELD DV II - 25°C - 12 rpm - module 3).

### Exemple 14

### Exemple d'utilisation de la composition de l'exemple 3 pour la préparation d'une crème

La phase lipophile (15 g d'isostéarate d'isostéaryle) et la phase hydrophile (81 g d'eau osmosée) qui contient 4 g de la composition à base de polyglycosides de l'exemple 3, sont chauffées séparément à une température de 70°C. La phase hydrophile est mise sous agitation (1500 rpm) et on y ajoute progressivement la phase lipophile. On laisse ensuite refroidir l'émulsion sous agitation lente (300 rpm) jusqu'à une température de l'ordre de 25°C.
Après 1 jour de mûrissement, la viscosité de l'émulsion ainsi formée est de 10500 cp (BROOKFIELD DV II - 25°C - 12 rpm - module 3).

### Exemple comparatif 15

### Indices de crèmage des émulsions préparées à partir des compositions à base de polyglycosides et d'alcools gras

### Préparation des émulsions

### Compositions :

- 3 % en poids par rapport au poids total de l'émulsion de compositions à base de polyglycosides et d'alcools gras
- 10 % en poids par rapport au poids total de l'émulsion d'huile de tournesol
- 0,5 % en poids par rapport au poids total de l'émulsion de conservateur (Phénonip® )
- eau osmosée qsp 100 % en poids.

### Protocole :

Les constituants sont pesés successivement dans un bêcher de forme basse et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée à mûrir 24 heures à 25°C avant son analyse.

L'indice de crèmage est défini comme le pourcentage en volume d'émulsion résiduelle après un traitement de déstabilisation par centrifugation. Les centrifugations (4080 g pendant 1 heure) sont identiques pour l'ensemble des essais.
Après centrifugation, l'indice de crèmage est le rapport du volume d'émulsion résiduelle sur le volume total d'émulsion initiale multiplié par 100.

Toutes les compositions à base de polyglycosides et d'alcools gras utilisées contiennent 55 % en poids par rapport au poids total des compositions, d'un mélange d'hexadécanol et d'octadécanol (50/50 ; poids/poids). Les polyglycosides sont constitués de polyhexosides (polyglucosides de l'exemple de synthèse 1) et/ou de polypentosides (polyxylosides de l'exemple de synthèse 2 et/ou polyarabinosides de l'exemple de synthèse 3). Les résultats sont résumés dans les tableaux suivants :

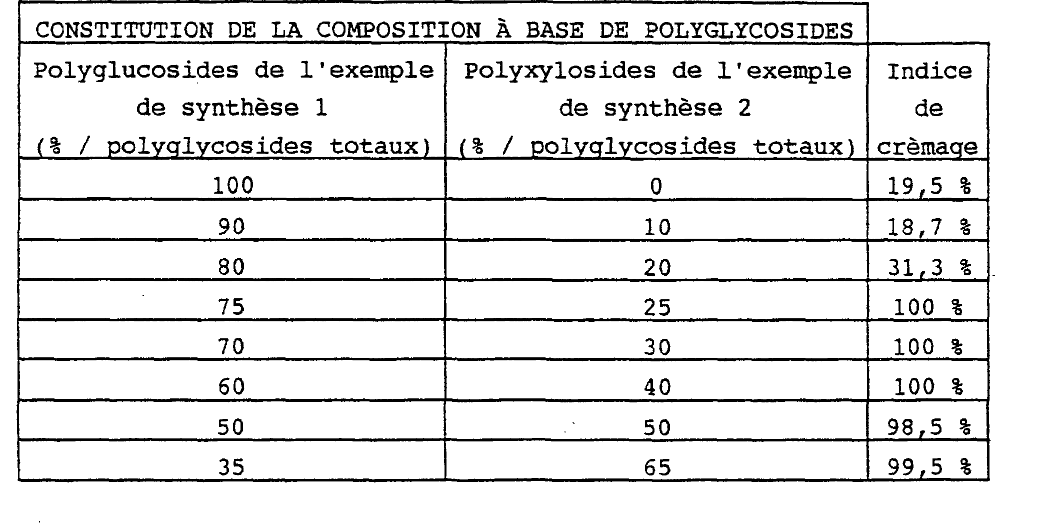

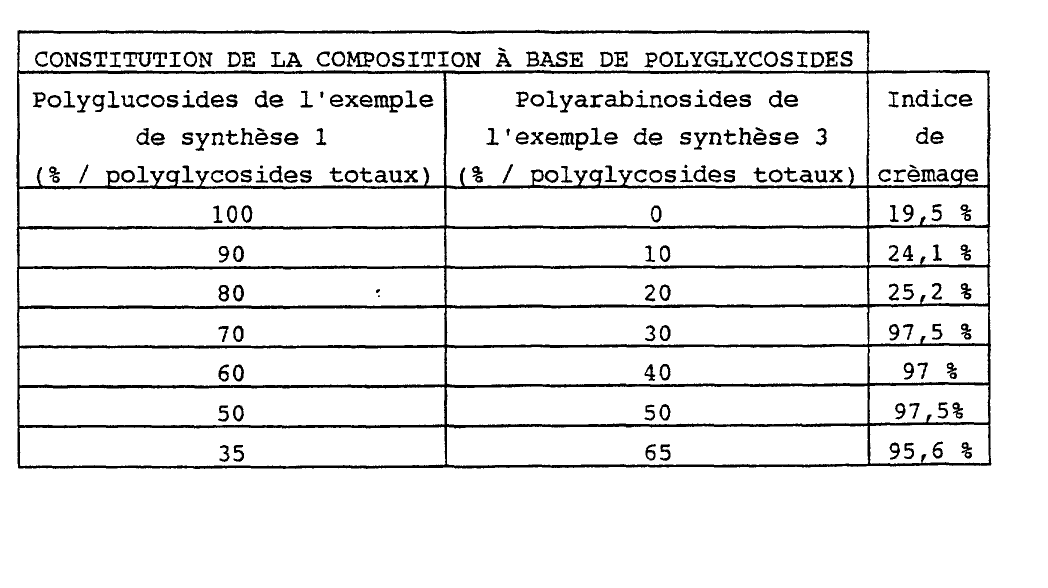

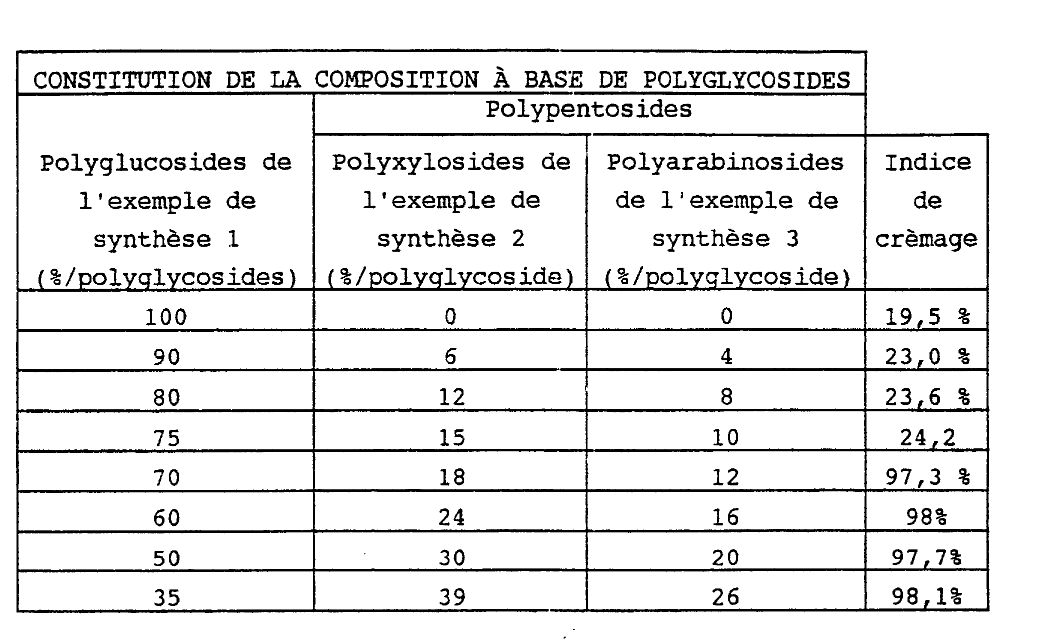

Les tableaux précédents permettent de mettre en évidence que les compositions à base de polyglycosides contenant de 25 à 65 % en poids par rapport aux poids total des polyglycosides, de polypentosides choisis parmi les polyarabinosides, les polyxylosides et leurs mélanges et de 35 à 75 % en poids de polyhexosides tels que les polyglucosides, permettent de former des émulsions présentant un indice de crémage proche de 100 %, c'est-à-dire stables à la centrifugation ; ce qui n'est pas le cas des compositions contenant exclusivement des polyhexosides tels que les polyglucosides de l'exemple de synthèse 1.

### Exemple comparatif 16

### Viscosité des émulsions préparées à partir des compositions à base de polyglycosides et d'alcools gras

Le protocole opératoire est celui de l'exemple précédent, si ce n'est qu'on mesure dans ce cas la viscosité des émulsions préparées à partir des compositions à base de polyglycosides. Les viscosités sont mesurées à 25°C à l'aide d'un viscosimètre BROOKFIELD DV II ( 12 rpm - module 3). Les résultats sont résumés dans les tableaux suivants :

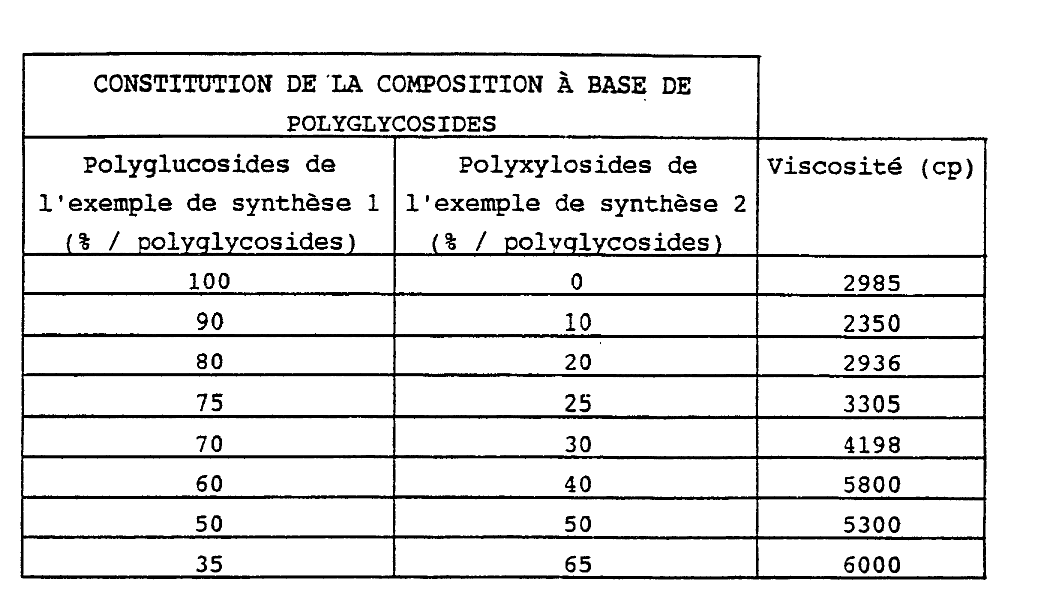

D'après le tableau les viscosités reflètent bien les valeurs d'indices de crèmage de l'exemple comparatif précédent. Globalement la viscosité des émulsions augmente avec l'augmentation du pourcentage de polyxylosides dans la composition émulsionnante. Comme une viscosité d'une émulsion élevée favorise sa stabilité future, cette constation montre bien que les compositions de l'invention à base de polyglycosides constitués de mélanges de polyhexosides et de polypentosides permettent la formation d'émulsions plus stables que celles constitués uniquement de polyglucosides sans qu'il soit nécessaire d'ajouter des épaississants.

### Exemple comparatif 17

### Comparaison des indices de crèmage des émulsions préparées à partir des compositions émulsionnantes de l'invention et à partir d'EMULGADE® PL 68/50

Cet exemple est réalisé de la même façon que l'exemple 15 précédent, si ce n'est que les polyglucosides de l'exemple de synthèse 1 sont remplacés par des polyglucosides commercialisés par HENKEL sous le nom d'EMULGADE® PL 68/50. Les résultats sont résumés dans les tableaux suivants :

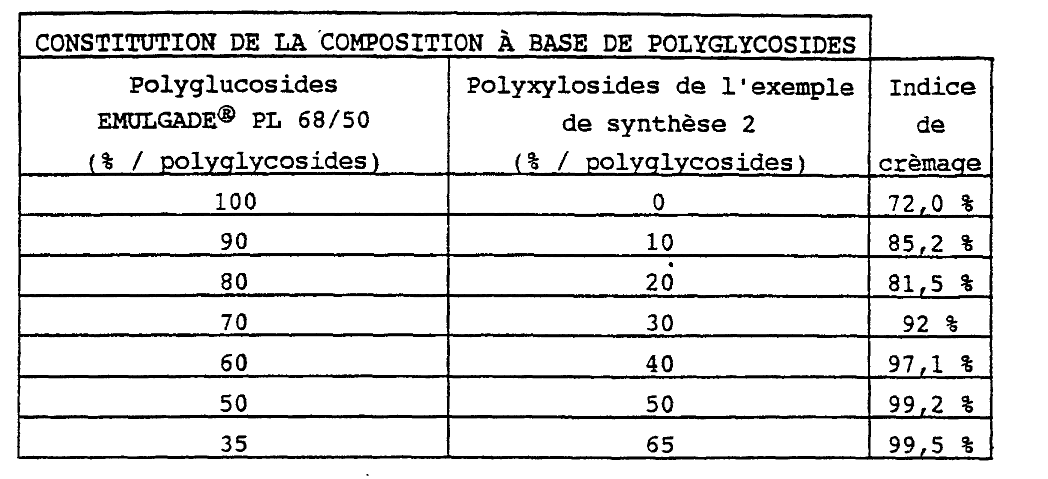

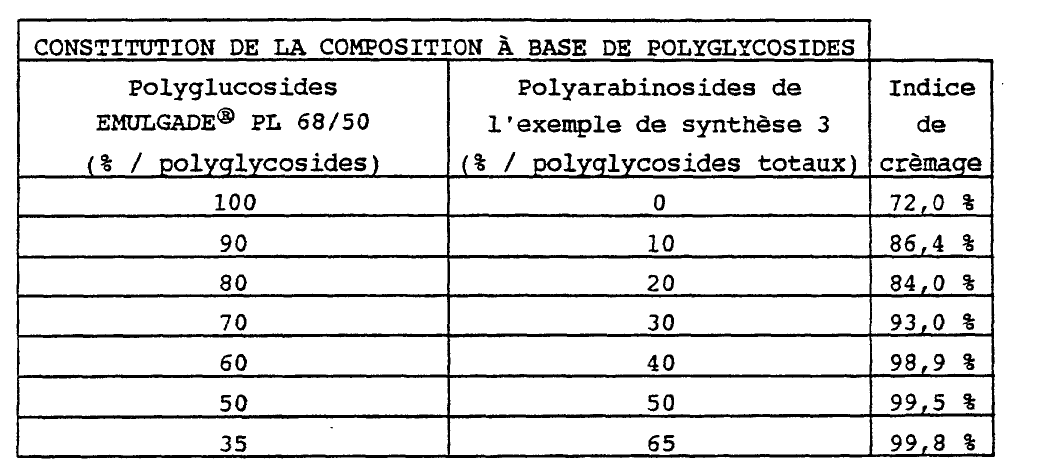

Les tableaux précédents permettent de mettre en évidence que les compositions à base de polyglycosides contenant des mélanges de polyglucosides et de polypentosides choisis parmi les polyarabinosides et les polyxylosides, permettent de former des émulsions présentant des indices de crémage plus élevés qu'à partir de compositions émulsionnantes contenant exclusivement des polyglucosides comme le produit EMULGADE® PL 68/50 commercialisé par HENKEL.

### Exemple comparatif 18

### Comparaison des viscosités des émulsions préparées à partir des compositions de l'invention et à partir d'EMULGADE® PL 68/50

Cet exemple est réalisé de la même façon que l'exemple 16 précédent, si ce n'est que les polyglucosides de l'exemple de synthèse 1 sont remplacés par des polyglucosides commercialisés par HENKEL sous le nom d'EMULGADE® PL 68/50. Les résultats sont résumés dans les tableaux suivants :

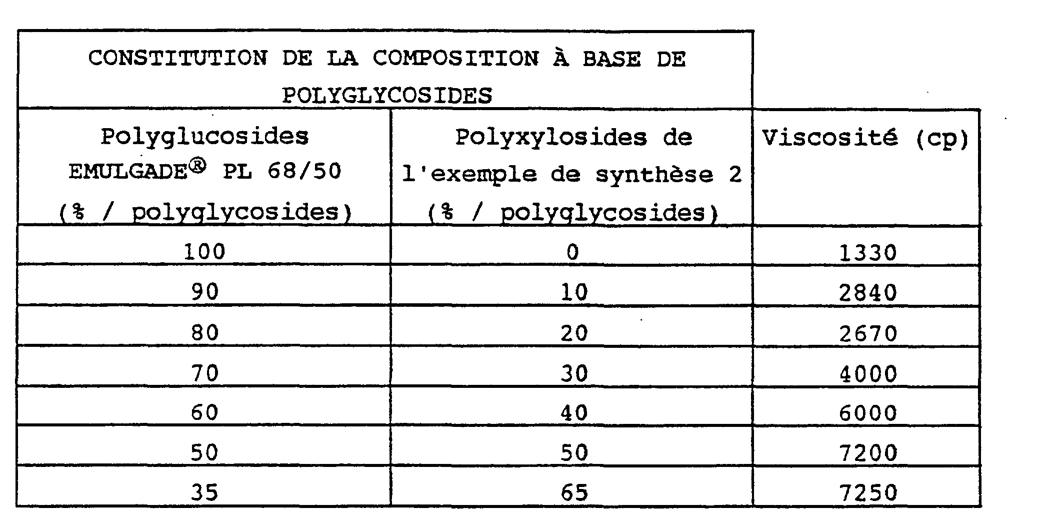

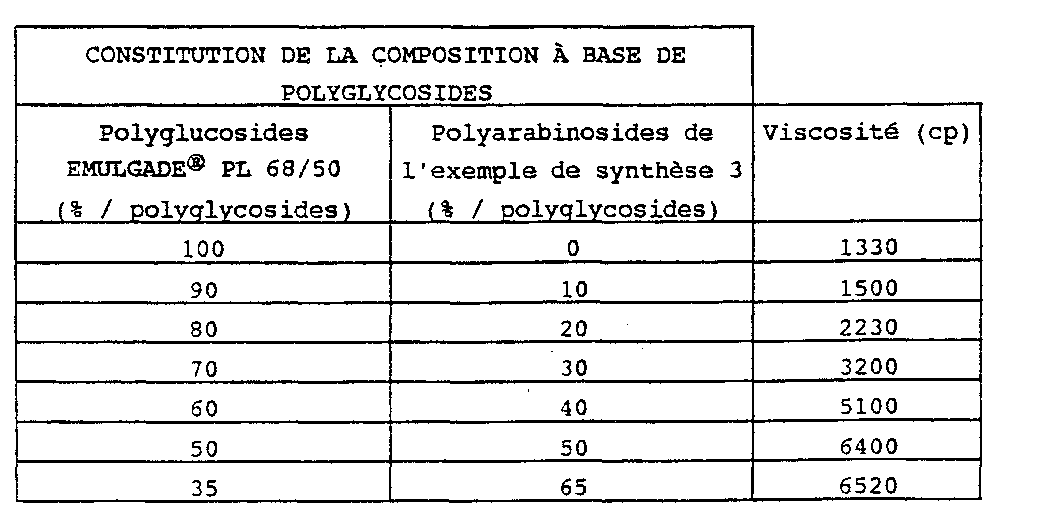

La viscosité des émulsions augmente avec l'augmentation du pourcentage de polyxylosides ou de polyarabinosides dans la composition émulsionnante EMULGADE® PL 68/50. Comme une viscosité d'une émulsion élevée favorise sa stabilité future, cette constation montre bien que les compositions de l'invention à base de polyglycosides constitués de mélanges de polyhexosides et de polypentosides permettent la formation d'émulsions plus stables que celles constitués uniquement de polyglucosides comme c'est le cas pour EMULGADE® PL 68/50.

### Exemple 19

### Stabilité des émulsions en fonction de la concentration en huile et de la concentration en composition à base de polyglycosides de l'invention

### Préparation des émulsions

### Compositions :

- 2,5 à 5 % en poids par rapport au poids total de l'émulsion de compositions à base de polyglycosides et d'alcools gras, des exemples 3, 5 et 7.
- 0 à 80 % en poids par rapport au poids total de l'émulsion d'huile de tournesol
- 0,5 % en poids par rapport au poids total de l'émulsion de conservateur (Phénonip® constitué de mélanges de méthyl/éthyl/butyl/propyl parabènes et de phénoxyéthanol commercialisé par NIPA)
- eau osmosée qsp 100 % en poids.

### Protocole :

Les constituants sont pesés successivement et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée vieillir en étuve à 45°C.

Une émulsion est considérée stable lorsqu'après 90 jours à 45°C, on n'observe pas de déphasage.

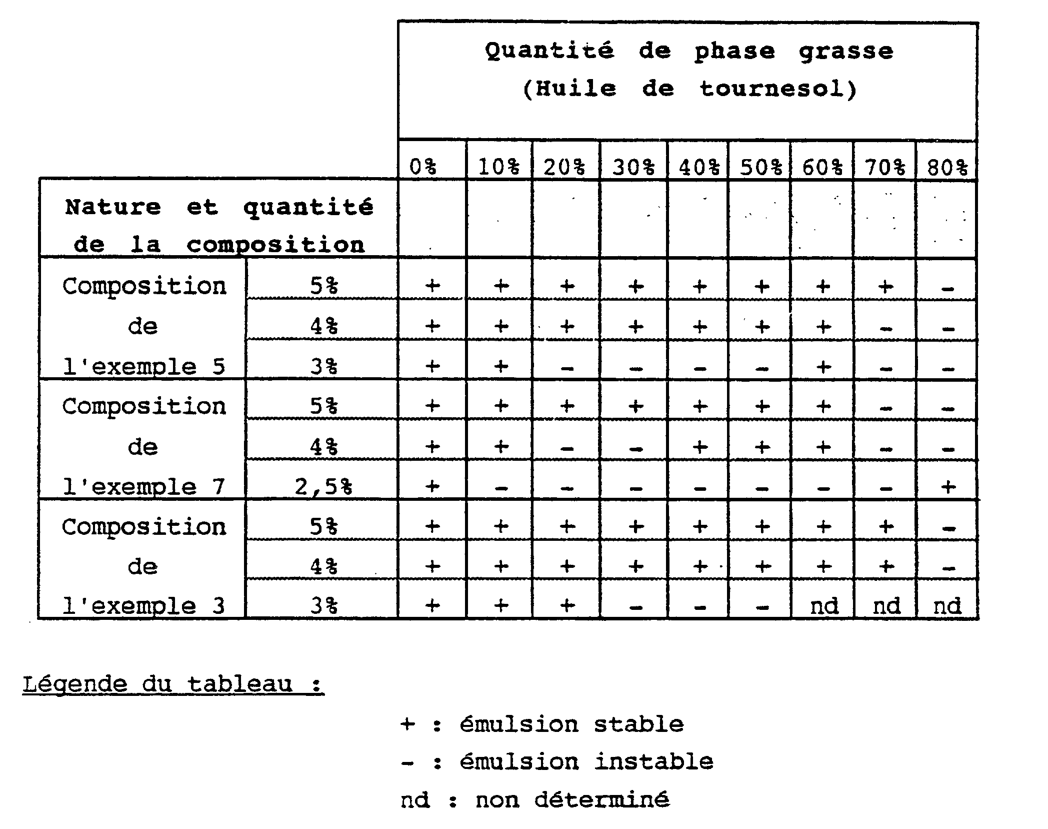

Doté d'un fort pouvoir émulsionnant, les compositions à base de polyglycosides de l'invention permettent d'obtenir des émulsions stables dès leur incorporation à de faibles teneurs (inférieures à 5 % en poids par rapport au poids total de l'émulsion), même en présence d'huile de tournesol réputée difficile à émulsionner.

### Exemple 20

### Stabilité des émulsions en fonction de la nature et de la concentration de la phase grasse

### Préparation des émulsions

### Compositions :

- 5 % en poids par rapport au poids total de l'émulsion de compositions à base de polyglycosides et d'alcools gras, des exemples 3, 5 et 7.
- 0 à 80 % en poids par rapport au poids total de l'émulsion de phase huileuse
- 0,5 % en poids par rapport au poids total de l'émulsion de conservateur (Phénonip®)
- eau osmosée qsp 100 % en poids.

Les phases huileuses utilisées sont les suivantes :
- Huile de tournesol
- Huile de paraffine fluide MARCOL 82 (ESSO)
- Triglycéride caprique/caprilique MIGLYOL 812N (HÜLS)

### Protocole:

Les constituants sont pesés successivement et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée vieillir en étuve à 45°C. Une émulsion est considérée stable lorsqu'après 90 jours à 45°C, on n'observe pas de déphasage.

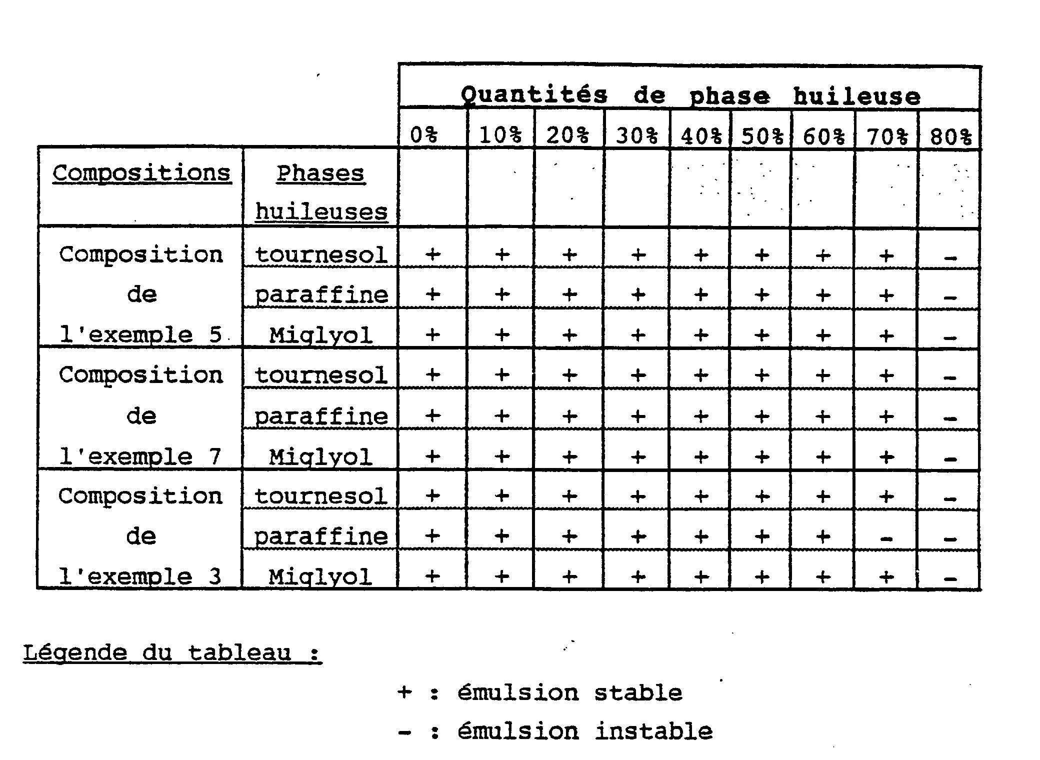

Avec l'ensemble des huiles testées, les compositions de l'invention permettent d'obtenir des émulsions très stables sur un large domaine de teneur en phase huileuse (0 à 70 % par rapport au poids total de l'émulsion).

### Exemple 21

### Influence de la teneur en phase grasse sur l'aspect des émulsions

### Préparation des émulsions

### Compositions :

- 2,5 à 5 % en poids par rapport au poids total de l'émulsion de compositions à base de polyglycosides et d'alcools gras, des exemples 3, 5 et 7.
- 0 à 70 % en poids par rapport au poids total de l'émulsion de phase huileuse (huile de tournesol)
- 0,5 % en poids par rapport au poids total de l'émulsion de conservateur (Phénonip®)
- eau osmosée qsp 100 % en poids.

### Protocole :

Les constituants sont pesés successivement et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée vieillir en étuve à 45°C. Une émulsion est considérée stable lorsqu'après 90 jours à 45°C, on n'observe pas de déphasage.

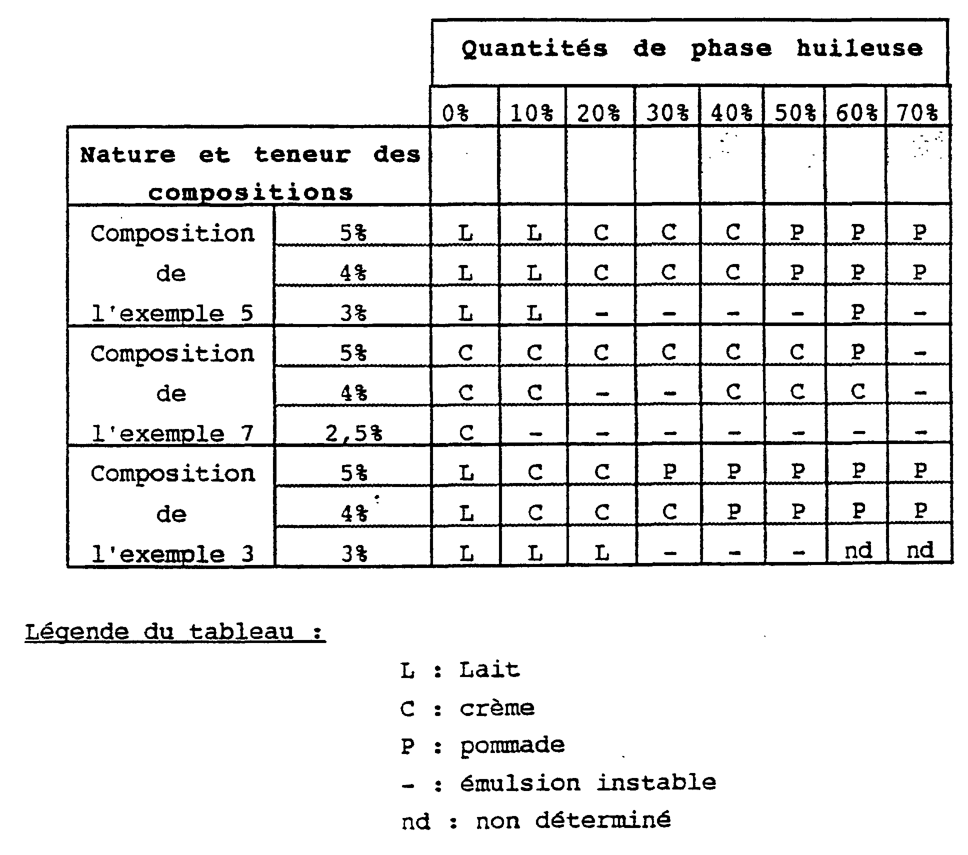

Quant à l'aspect des émulsions, nous constatons que les compositions de l'invention permettent de réaliser des émulsions parfaitement stables sans l'apport d'agents épaississants ou d'additifs spécifiques tout en couvrant à la fois les laits, les crèmes et les pommades.

### Exemple 22

### Exemple comparatif Stabilité des émulsions en fonction de la quantité de phase grasse et de la teneur en composition émulsionnante

### Préparation des émulsions

### Compositions :

- 2,5 å 5 % en poids par rapport au poids total de l'émulsion de compositions à base de polyglycosides et d'alcools gras, des exemples 3, 5 et 7 ou d'autres compositions du marché.
- 0 à 80 % en poids par rapport au poids total de l'émulsion de phase huileuse (huile de tournesol)
- 0,5 % en poids par rapport au poids total de l'émulsion de conservateur (Phénonip®)
- eau osmosée qsp 100 % en poids.
Les compositions du marché sont les suivantes :
- Composition à base de polyosides d'alkyle et d'alcool gras : MONTANOV® 68 (SEPPIC)
- PEG palmitostearate SE TEFOSE® 1500 (GATTEFOSSE)
- Cire d'abeilles SE APIFIL® (GATTEFOSSE)
- Glyceryl stearate SE CUTINA® (HENKEL)
- Composition à base de tensioactifs polyoxyéthylénés et d'alcools gras : SINNOWAX AOE (HENKEL)

### Protocole :

Les constituants sont pesés successivement et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée vieillir en étuve à 45°C. Une émulsion est considérée stable lorsqu'après 90 jours à 45°C, on n'observe pas de déphasage.

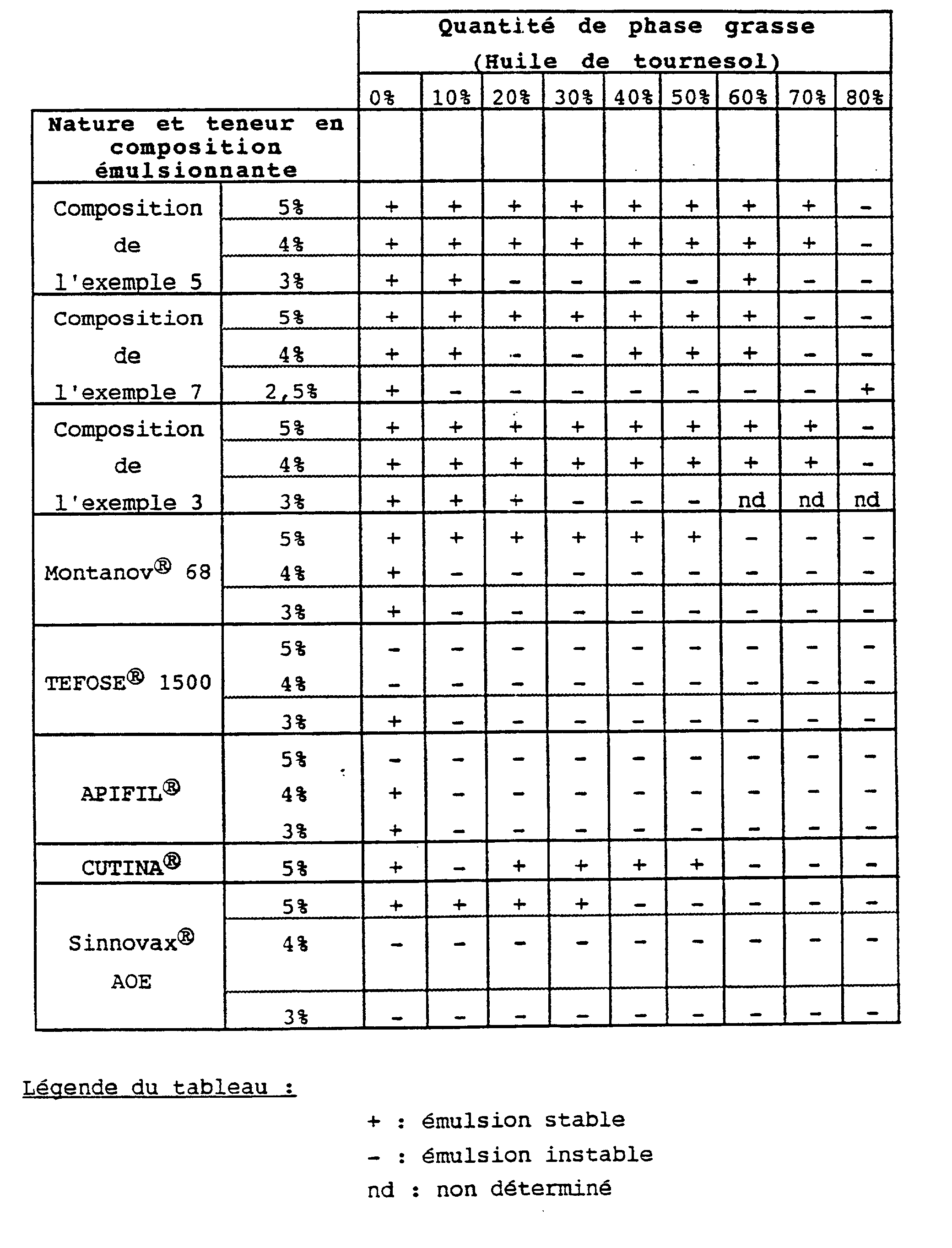

Le tableau précédent permet de montrer que les compositions de l'invention présentent des domaines de stabilité des émulsions largement supérieurs à ceux obtenus avec des compositions concurrentes. Par rapport à la composition MONTANOV® 68 constituée de polyglucosides et d'alcool cétéarylique, les compositions de l'invention constituées de mélanges de polyhexosides et de polypentosides possèdent un domaine de stabilité plus large notamment pour de faibles concentrations en agent émulsionnant.

### Exemple 23

### Influence de la teneur en alcools gras des compositions de l'invention sur la stabilité des émulsions préparées à partir de ces compositions

### Préparation des émulsions

### Compositions :

- 2,5 à 5 % en poids par rapport au poids total de l'émulsion de compositions à base de polyglycosides et d'alcools gras contenant différentes teneurs en alcool gras.
- 0 à 80 % en poids par rapport au poids total de l'émulsion de phase huileuse (huile dé tournesol)
- 0,5 % en poids par rapport au poids total de l'émulsion de conservateur (Phénonip®)
- eau osmosée qsp 100 % en poids.

### Compositions utilisées:

A - Composition de l'exemple 7 contenant 60 % en poids d'alcool cétéarylique (hexadécanol/octadécanol 50/50) par rapport au poids total de la composition.
B - Composition de l'exemple 7 dans laquelle de l'alcool cétéarylique (hexadécanol/octadécanol 50/50) a été ajouté de telle sorte qu'elle contienne 75 % en poids d'alcool cétéarylique par rapport au poids total de la composition.
C - Composition de l'exemple 7 dans laquelle de l'alcool cétéarylique (hexadécanol/octadécanol 50/50) a été ajouté de telle sorte qu'elle contienne 90 % en poids d'alcool cétéarylique par rapport au poids total de la composition.

### Protocole :

Les constituants sont pesés successivement et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée vieillir en étuve à 45°C. Une émulsion est considérée stable lorsqu'après 90 jours à 45°C, on n'observe pas de déphasage.

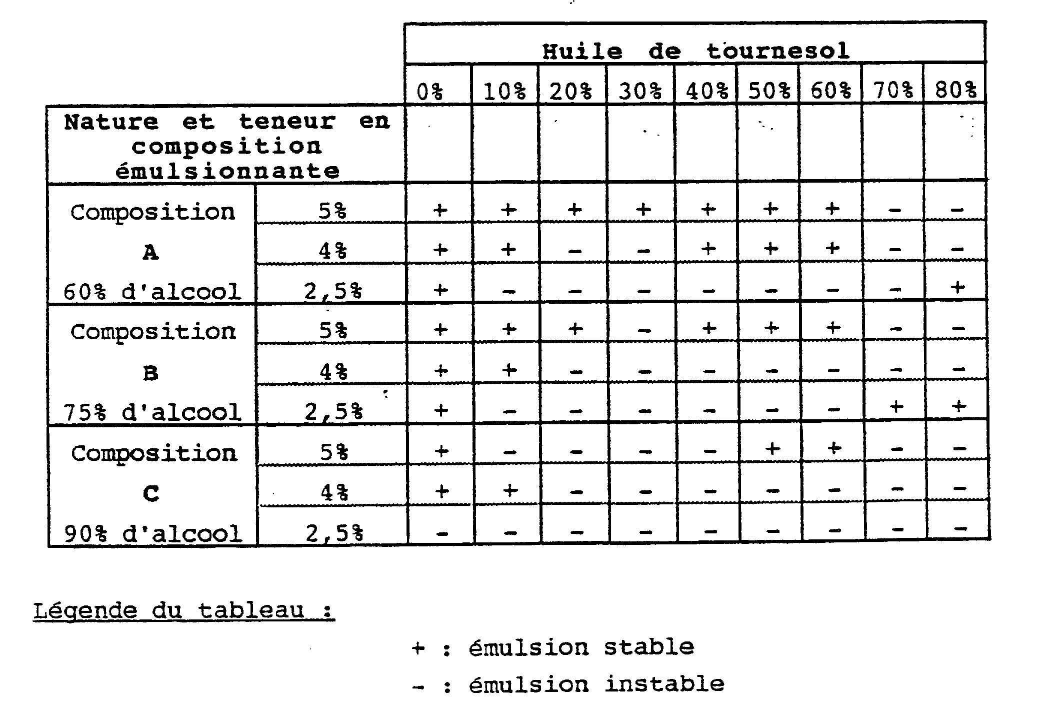

Le tableau précédent permet de mettre en évidence, que quand la teneur en alcool gras s'élève dans les compositions, ces dernières deviennent moins efficaces et que les domaines de stabilité des émulsions formées sont plus restreints. C'est pourquoi les compositions à base de polyglycosides et d'alcools gras de la présente invention contiennent au maximum 65 % en poids par rapport au poids total des compositions, d'alcools gras libres.

### Exemple 24

### Exemple comparatif Viscosité des émulsions en fonction de la teneur en chlorure de sodium

Les émulsions de cet exemple sont réalisés de la façon suivante :
- Chauffer séparément 10 minutes à 75°C la phase grasse et la phase aqueuse.
   La phase grasse comprend 4 % en poids par rapport au poids total de l'émulsion, de composition émulsionnante de l'exemple 1 ou de composition EMULGADE® PL 68/50, 10 % d'isostéarate d'isostéaryle et 0,5 % de conservateur (Phénonip®).
   La phase aqueuse comprend 0 à 2% par rapport au poids total de la composition émulsionnante de chlorure de sodium et l'eau (qsp 100 % en poids par rapport au poids total de l'émulsion).
- La phase aqueuse est ajoutée à la phase huileuse à 75 °C sous agitation (1300 rpm) et on laisse agiter à cette vitesse pendant 5 minutes.
- L'agitation est poursuivie à l'aide d'un barreau magnétique (260 rpm) pendant 15 minutes jusqu'au refroidissement de l'émulsion. L'émulsion est laissée vieillir à 25°C pendant 8 jours et sa viscosité est mesurée sur un viscosimètre BROOKFIELD DV II , module 3, 12 rpm et à 25°C.
Les résultats sont résumés dans le tableau suivant :

| Teneur en NaCl | Viscosité de l'émulsion préparée à partir de la composition de l'exemple 1 | Viscosité de l'émulsion préparée à partir de la composition EMULGADE® PL 68/50 |
|---|---|---|
| 0 % | 30000 cp | 32000 cp |
| 0,25 % | 26000 cp | 22000 cp |
| 0,5 % | 29000 cp | 17000 cp |
| 0,75 % | 34000 cp | 16000 cp |
| 1 % | 33000 cp | 17500 cp |
| 2 % | 35000 cp | 14000 cp |

Le tableau comparatif précédent permet de montrer que la composition à base de polyglycosides de l'exemple 1 de la présente invention présente une faible sensibilité au chlorure de sodium par rapport à la composition EMULGADE® PL 68/50. Ainsi, la viscosité des émulsions formées est a peu près constante quelle que soit la quantité de chlorure de sodium.

### Exemple 25

| **Crème auto-bronzante et hydratante résistante à l'eau** | | |
|---|---|---|
| A - | Composition de l'exemple 8 | 4,0 % |
| | Aloe vera | 1,0 % |
| | Beurre de karité | 0,2 % |
| | Diméthicone | 2,0 % |
| | 2-octyl dodécyl myristate (MOD) | 3,0 % |
| | Propylglycol stéarate (Stepan PGMS) | 1,0 % |
| | Acide stéarique | 1,0 % |
| | Vitamine E | 0,1 % |
| | Acide hyaluronique (VITALHYAL) | 1,0 % |
| | Phénonip | 0,5 % |
| | Eau QSP | 100 % |
| | | |
| B - | Carbopol Ultrez 10 (BF Goodrich) | 0,15 % |
| | Eau | 14,85 % |
| | | |
| C - | Dihydroxyacétone | 5,0 % |
| | Eau | 10,0 % |
| | | |
| D - | Fragrance | QS |

### Procédé de fabrication de la crème :

Peser tous les ingrédients de A
Peser tous les ingrédients de B et faire une dispersion
Additionner B dans A
Chauffer à 75 °C
Mélanger à 2000 rpm quelques minutes à 75 °C
Laisser refroidir à 30°C en agitant à 300 rpm
Préparer la solution C à température ambiante
Additionner C et D dans l'émulsion
Corriger le pH si cela est nécessaire.

### Exemple 26

| **Lait hydratant** | |
|---|---|
| Composition de l'exemple 5 | 2,0 % |
| Miglyol 812 N | 3,0 % |
| Isostéarate d'isostéaryle | 3,0 % |
| Diméthicone | 2,0 % |
| Alcool stéarylique | 1,0 % |
| Acide hyaluronique (VITALHYAL) | 1,0 % |
| Phénonip | 0,5 % |
| Eau QSP | 100 % |

### Procédé de fabrication du lait:

Peser tous les ingrédients
Chauffer à 75 °C
Mélanger à 3000 rpm quelques minutes à 75 °C
Laisser refroidir à 30°C en agitant à 500 rpm
Corriger le pH si cela est nécessaire.

### Exemple 27

| **Crème de nuit** | |
|---|---|
| Composition de l'exemple 9 | 4,0 % |
| Adipate diisopropyl | 5,0 % |
| Oléyl érucate (Cétiol J600) | 1,5 % |
| Huile de jojoba | 1,5 % |
| Huile de camélia | 1,5 % |
| Huile d'amandes douces | 0,5 % |
| Hexanol (SIPOL C16) | 2,0 % |
| Acide hyaluronique (VITALHYAL) | 2,0 % |
| Hydrolysat de protéines de blé | 0,5 % |
| Vitamine E | 0,1 % |
| Phénonip | 0,5 % |
| Fragrance | QS |
| Eau QSP | 100 % |

### Procédé de fabrication de la crème :

Peser tous les ingrédients excepté la fragrance
Chauffer à 75 °C
Mélanger à 3000 rpm quelques minutes à 75 °C
Laisser refroidir à 30°C en agitant à 300 rpm
Additionner la fragrance
Stopper l'agitation
Corriger le pH si cela est nécessaire.

### Exemple 28

| **Crème** | |
|---|---|
| Composition de l'exemple 5 | 4,0 % |
| Huile d'amandes douces | 2,0 % |
| Miglyol 812 N | 2,0 % |
| Isostéarate d'isostéaryle | 2,0 % |
| Huile de paraffine (MARCOL 82) | 2,0 % |
| Diméthicone | 2,0 % |
| Octadécanol (Steraffine) | 1,0 % |
| Vitamine E | 0,2 % |
| Phénonip | 0,5 % |
| Fragrance | QS |
| Eau QSP | 100 % |

### Procédé de fabrication de la crème :

Peser tous les ingrédients excepté la fragrance
Chauffer à 75 °C
Mélanger à 3000 rpm quelques minutes à 75 °C
Laisser refroidir à 30°C en agitant à 300 rpm
Additionner la fragrance
Stopper l'agitation
Corriger le pH si cela est nécessaire.

### Exemple 29

| **Lait démaquillant** | |
|---|---|
| Composition de l'exemple 2 | 2,0 % |
| Huile d'amandes douces | 2,0 % |
| Miglyol 812 N | 2,0 % |
| Isostéarate d'isostéaryle | 2,0 % |
| Propylglycol stéarate ( Stepan PGMS) | 1,0 % |
| Diméthicone | 2,0 % |
| Hexanol (SIPOL C16) | 1,0 % |
| Sophoroses lipides (SOPHOLIANCE) | 2,0 % |
| Hydrolysat de protéines de blé | 0,2 % |
| Vitamine E | 0,2 % |
| Phénonip | 0,5 % |
| Fragrance | QS |
| Eau QSP | 100 % |

### Procédé de fabrication du lait :

Peser tous les ingrédients excepté la fragrance
Chauffer à 75 °C
Mélanger à 3000 rpm quelques minutes à 75 °C
Laisser refroidir à 30°C en agitant à 300 rpm
Additionner la fragrance
Stopper l'agitation
Corriger le pH si cela est nécessaire.

### Exemple 30

| **Crème solaire** | | |
|---|---|---|
| A. | Composition de l'exemple 5 | 3,0 % |
| | Huile de tournesol | 2 % |
| | Miglyol 812 N | 1 % |
| | Huile de paraffine | 3 % |
| | Phénonip | 0,5 % |
| | Vitamine E | 0,1 % |
| | Eau QSP | 100 % |
| | | |
| B. | Dioxyde de titane | 3,0 % |
| | Diméthicone | 5,0 % |
| | | |
| C. | DHA | 5,0 % |
| | Eau | 10,0 % |

### Procédé

- peser les ingrédients de A
- chauffer à 50°C
- mélanger B intimement
- agiter A à 2000 rpm quelques minutes à 50°C
- ajouter B en maintenant l'agitation quelques minutes
- ralentir l'agitation à 2/300 rpm jusqu'à 30/35°C
- ajuter C lorsque la température est inférieure à 40°C
- rectifier le pH (entre 4 et6)

### Exemple 31

| **Crème contenant des sophoroses lipides** | |
|---|---|
| Composition de l'exemple 5 | 4,0 % |
| Huile de paraffine (MARCOL 82) | 2,0 % |
| Miglyol 812 N | 3,0 % |
| Isostéarate d'isostéaryle | 3,0 % |
| Diméthicone | 2,0 % |
| Octadécanol (Stéraffine) | 2,0 % |
| Sophoroses lipides (SOPHOLIANCE) | 1,0 % |
| Phénonip | 0,5 % |
| Eau QSP | 100 % |

### Procédé de fabrication de la crème :

Préparer une solution aqueuse limpide de SOPHOLIANCE à 25 % de matière sèche, à pH 6 (NaOH).
Peser tous les ingrédients excepté SOPHOLIANCE
Chauffer à 75 °C pendant 10 minutes
Mélanger à 1500 rpm 1 minute à 75 °C
Laisser refroidir en agitant à 300 rpm
Additionner SOPHOLIANCE vers 50°C
Corriger le pH si cela est nécessaire.
Stopper l'agitation vers 30°C

### Exemple 32

### Crème hydratante pour les mains

### * Particularité

Crème H/E hydratante, non collante, procurant une sensation de fraîcheur pour les mains et de texture favorable à l'utilisation par les peaux grasses.

### * Formulation

| *Excipients* | *Pourcentages* |
|---|---|
| 1 - Arlamol S7 (silicone) | 11 % |
| 2 - Composition de l'exemple 5 | 4 % |
| 3 - Composition de l'exemple 4 | 1 % |
| 4 - Glycérine | 4 % |
| 5 - Conservateur - (Phénonip) | 0,5 % |
| 6 - Antioxydant | 0,1 % |
| 7 - Eau | qsp 100 % |

### * Mode opératoire

- Chauffer à 70° C, sous agitation, les excipients 1,2,3,4,5 et 7,
- Ajouter 6 à température ambiante.

### Exemple 33

### Crème hydratante pour bébés et peaux sèches

### * Particularité

Crème hydratante H/E non collante et facile d'étalement pour bébés et peaux sèches.

### * Formulation

| *Excipients* | *Pourcentages* |
|---|---|
| 1 - Vaseline | 7,5 |
| 2 - Composition de l'exemple 5 | 5 |
| 3 - Miglyol 812 N | 3,5 |
| 4 - Myristate d'isopropyle | 2 |
| 5 - Huile de calandula | 1 |
| 6 - Huile d'avocat | 1 |
| 7 - Phénonip | 0,5 |
| 8 - Antioxydant | 0,1 |
| 9 - Eau | qsp100 |

### * Mode opératoire.

- Chauffer à 70° C, sous agitation, les excipients 1 à 7 et 9,
- Ajouter 8 à température ambiante.

### Exemple 34

### Lait hydratant riche en huiles végétales

### * Particularité

Lait H/E très hydratant, non collant, riche en huiles végétales et facile à étaler.

### * Formulation

| *Excipients* | *Pourcentages* |
|---|---|
| 1 - Miglyol 812 N | 3 |
| 2 - Myristate d'isopropyle | 3 |
| 3 - Composition de l'exemple 5 | 3 |
| 4 - Huile de noisette | 2 |
| 5 - Huile d'abricot | 2 |
| 6 - Epaississant (Méthocel 40-202-E) | 1 |
| 7 - Conservateur (Phénonip) | 0,5 |
| 8 - Antioxydant | 0,1 |
| 9 - Eau | qsp100 |

### * Mode opératoire

- Chauffer à 70° C, sous agitation, tous les excipients sauf 8,
- Ajouter 8 à température ambiante.

### Exemple 35

### Lait solaire

### * Particularité

Lait solaire H/E non collant et photostable, anti IR, UV_{A}, UV_{B}, et UV_{C}.

### * Formulation

| *Excipients* | *Pourcentages* |
|---|---|
| 1 - Filtre UV_{A}, UV_{B} et UV_{C} (Covabsorb) | 8 |
| 2 - Miglyol 812 N | 3 |
| 3 - Myristate d'isopropyle | 3 |
| 4 - Composition de l'exemple 5 | 2 |
| 5 - Composition de l'exemple 6 | 1 |
| 6 - Huile de noisette | 2 |
| 7 - Huile d'abricot | 2 |
| 8 - Filtre IR (dioxyde de titane) | 2 |
| 9 - Epaississant (Méthocel 40-202-E) | 1 |
| 10 - Conservateur (Phénonip) | 0,6 |
| 11 Eau | qsp100 |

### * Mode opératoire

- Chauffer à 70° C, sous agitation, tous les excipients,
- Rectifier le pH si nécessaire.

## Revendications

1. Composition à base de polyglycosides qui comprend de 30 à 65 % en poids d'au moins un alcool gras de formule ROH, où R est un radical aliphatique, saturé ou insaturé ayant de 1 à 4 insaturations éthyléniques, linéaire ou ramifié, ayant de 12 à 22 atomes de carbone, le reste étant choisi, sauf des impuretés, parmi :
- un mélange de polyglycosides comprenant de 35 à 75 % en poids, par rapport aux polyglycosides, de polyhexosides de formule
R¹O(H)ₙ₁
dans laquelle R¹ est un radical aliphatique, saturé ou insaturé ayant de 1 à 4 insaturations éthyléniques, linéaire ou ramifié, ayant de 12 à 22 atomes de carbone, H est le reste d'un hexose, ni est compris entre 1 et 5 ; et de 25 à 65 % en poids par rapport aux polyglycosides d'au moins un polypentoside de formule :
R²O(P)ₙ₂
dans laquelle R² est un radical aliphatique, saturé ou insaturé ayant de 1 à 4 insaturations éthyléniques, linéaire ou ramifié, ayant de 12 à 22 atomes de carbone, P est le reste d'un pentose choisi parmi l'arabinose et le xylose, n2 est compris entre 1 et 5 ;
- et un mélange de polyglycosides de formule:
R³O(G₁) ₐ (G₂) _{b} (G₃) _{c} (G₄) _{d} (G₅)ₑ
dans laquelle R³ est un radical aliphatique, saturé ou insaturé ayant de 1 à 4 insaturations éthyléniques, linéaire ou ramifié, ayant de 12 à 22 atomes de carbone, G₁, G₂, G₃, G₄, G₅ sont indépendamment les uns des autres, des restes d'un ose choisi parmi les hexoses et les pentoses, ces derniers étant choisis parmi l'arabinose et le xylose, les hexoses représentant 35 à 75 % en poids de l'ensemble des restes d'oses G₁, G₂, G₃, G₄ et G₅ et les pentoses de 25 à 75 % en poids de l'ensemble des restes d'oses G₁, G₂, G₃, G₄ et G₅ ; a, b, c, d et e étant égaux à 0 ou à 1, la somme de a, b, c, d et e étant au moins égale à 1.

2. Composition selon la revendication 1, dans laquelle les polyhexosides ou les restes d'hexoses sont constitués respectivement par au moins 80 % de polyglucosides ou de restes de glucose.

3. Composition selon la revendication 1 qui comprend des polyglycosides dont les radicaux R¹ et R² ou le radical R³ sont identiques au radical R de l'alcool gras.

4. Composition selon la revendication 1, dans laquelle l'alcool gras présente de 14 à 22 atomes de carbone.

5. Composition selon la revendication 4, dans laquelle l'alcool gras est un mélange d'alcools ayant de 16 à 18 atomes de carbone.

6. Composition selon la revendication 1 qui comprend de 40 à 60 % d'alcool gras et de préférence de 52 à 57 % d'alcool gras.

7. Composition selon la revendication 1 qui comprend un mélange de 45 à 70 % en poids par rapport aux polyglycosides, de polyhexosides de formule R¹O(H)ₙ₁ et de 30 à 55 % en poids de polypentosides de formule R²O(P)ₙ₂.

8. Composition suivant la revendication 1 qui comprend des polyglycosides de formule :
R³O(G₁)ₐ(G₂)_{b}(G₃)_{c}(G₄)_{d}(G₅)ₑ
dans laquelle les restes d'hexoses représentent 45 à 70 % en poids de l'ensemble des restes d'oses G₁, G₂, G₃, G₄ et G₅ et les restes de pentoses 30 à 55 % en poids de l'ensemble des restes d'oses G₁, G₂, G₃, G₄ et G₅.

9. Composition selon la revendication 1, dans laquelle les polyglycosides sont dérivés de sirops de sucres obtenus par hydrolyse de co-produits d'origine végétale riches en amidon et en hémicellulose.

10. Composition selon la revendication 1, dans laquelle les polyglycosides sont dérivés de sons de blé, de mélanges d'amidon et de sons de blé, de sons de maïs, de drèches de maïs ou de leurs mélanges.

11. Procédé de préparation d'une émulsion comprenant la mise en presence :
a) de 2 à 60 % en poids d'une phase huileuse ;
b) de 1 à 10 % en poids d'une composition à base de polyglycosides selon la revendication 1
c) le reste choisi parmi une phase aqueuse ou une phase aqueuse additionnée d'une composition émulsionnante autre que celle mentionnée sous b).

12. Procédé selon la revendication 11, dans lequel la phase huileuse de l'émulsion contient au moins 50 % en poids par rapport au poids total de la phase huileuse, d'huiles végétales comprenant au moins 40 % de composés choisis parmi les triglycérides d'acide linoléique, les huiles de silicone.

13. Procédé selon la revendication 11, dans lequel la phase huileuse représente de 10 à 40 % en poids par rapport au poids total de l'émulsion.

14. Procédé selon la revendication 12, dans lequel la composition b) représente de 3 à 4 % en poids du poids total de l'émulsion.

## Patentansprüche

1. Zusammensetzung auf Basis von Polyglycosiden, die 30 bis 65 Gew.% mindestens eines Fettalkohols der Formel ROH, wobei R einen aliphatisches Radikal, gesättigt oder ungesättigt mit 1 - 4 ethylenischen Doppelbindungen, linear oder verzweigt, mit 12 - 22 Kohlenstoffatomen, ist, wobei der Rest ausgewählt ist - ausgenommen Verunreinigungen, aus:
- einer Mischung von Polyglycosiden, die 35 - 75 Gew.% bezogen auf Polyglycoside, Polyhexosen der Formel R¹O(H)ₙ₁
aufweist, wobei R¹ ein aliphatisches Radikal, gesättigt oder ungesättigt mit 1 - 4 ethylenischen Doppelbindungen, linear oder verzweigt, mit 12 - 22 Kohlenstoffatomen, ist, H der Rest einer Hexose, n1 zwischen 1 und 5 liegt; und 25
- 65 Gew.% bezogen auf Polyglycoside mindestens eines Polypentosids der Formel:
R²O(P)ₙ₂
wobei R² ein aliphatischer Rest, gesättigt oder ungesättigt mit 1 - 4 ethylenischen Doppelbindungen, linear oder verzweigt mit 12 - 22 Kohlenstoffatomen, P der Rest einer Pentose, ausgewählt aus Arabinose und Xylose, ist, n2 zwischen 1 und 5 liegt;
- und eine Mischung von Polyglycosiden der Formel:
R³O(G₁)ₐ(G₂)_{b}(G₃)_{c}(G₄)_{d}(G₅)ₑ
wobei R³ ein aliphatisches Radikal, gesättigt oder ungesättigt mit 1 - 4 ethylenischen Doppelbindungen ist, geradkettig oder verzweigt, mit 12 - 22 Kohlenstoffatomen; G₁, G₂, G₃, G₄, G₅ unabhängig voneinander sind und Reste einer Ose sind, ausgewählt aus Hexosen und Pentosen, wobei letztere ausgewählt sind aus Arabinose und Xylose, wobei die Hexosen 35 - 75 Gew.% der Gesamtmenge der Reste der Osen ; G₁, G₂, G₃, G₄ und G₅ repräsentieren und Pentosen 25 - 75 Gew.% des gesamten Restes der Osen G₁, G₂, G₃, G₄ und G₅; a, b, c, d und e gleich 0 oder 1 sind, wobei die Summe von a, b, c, d und e mindestens gleich 1 ist.

2. Zusammensetzung nach Anspruch 1, wobei die Polyhexosiden oder die Hexose-Reste jeweils aus mindestens 80 % Polyglycosiden oder Resten von Glucose bestehen.

3. Zusammensetzung nach Anspruch 1, die Polyglycoside aufweist, wobei die Radikale R¹ und R² oder das Radikal R³ identisch mit dem Radikal R des Fettalkohols sind.

4. Zusammensetzung nach Anspruch 1, wobei der Fettalkohol 14 - 22 Kohlenstoffatome aufweist.

5. Zusammensetzung nach Anspruch 4, wobei der Fettalkohol eine Mischung von Alkoholen mit 16 - 18 Kohlenstoffatomen ist.

6. Zusammensetzung nach Anspruch 1, die 40 - 60 Gew.% Fettalkohol und bevorzugt 52 - 57 Gew.% Fettalkohol aufweist.

7. Zusammensetzung nach Anspruch 1, die eine Mischung von 45 - 70 Gew.% bezogen auf Polyglycoside, Polyhexosen der Formel R¹O(H)ₙ₁ aufweist und 30 - 55 Gew.% von Polypentosiden der Formel R²O(P)ₙ₂.

8. Zusammensetzung nach Anspruch 1, die Polyglycoside der Formel:
R³O(G₁)ₐ(G₂)_{b}(G₃)_{c}(G₄)_{d}(G₅)ₑ
aufweist, wobei die Reste Hexosen 45 - 70 Gew.% der Gesamtmenge der Reste der Osen G₁, G₂, G₃, G₄ und G₅ stellen und die Reste der Pentosen 30 - 55 Gew.% der Gesamtmenge der Reste der Osen G₁, G₂, G₃, G₄ und G₅.

9. Zusammensetzung nach Anspruch 1, wobei die Polyglycoside von Zuckersirupen abgeleitet sind, erhalten durch Hydrolyse der Nebenprodukte vegetabilen Ursprungs, reich an Stärke und Hemicellulose.

10. Zusammensetzung nach Anspruch 1, wobei die Polyglycoside Abkömmlinge der Weizenkleie, von Mischungen von Stärke und Weizenkleie, von Maiskleie, von Trebern von Mais oder ihre Mischungen sind.

11. Verfahren zur Herstellung der Zusammensetzung einer Emulsion, wobei zusammengefaßt werden:
a) 2 - 60 Gew.% einer öligen Phase
b) 1- 10 Gew.% einer Zusammensetzung auf Basis von Polyglycosiden nach Anspruch 1,
c) wobei der Rest ausgewählt ist aus einer wässrigen Phase oder einer wässrigen Phase mit Zugabe einer emulsionsbildenden Zusammensetzung, unterschiedlich zu der unter b) genannten.

12. Verfahren nach Anspruch 11, wobei die ölige Phase der Emulsion mindestens 50 Gew.% bezogen auf das Gesamtgewicht der öligen Phase Pflanzenöle beinhaltet, wobei mindestens 40 Gew.% der Zusammensetzung ausgewählt sind aus Triglyceriden der Linofeinsäüre, Siloconölen.

13. Verfahren nach Anspruch 11, wobei die ölige Phase 14 - 40 Gew.% bezogen auf das Gesamtgewicht der Emulsion, repräsentiert.

14. Verfahren nach Anspruch 12, wobei die Zusammensetzung b) 3 - 4 Gew.% des Gesamtgewichts der Emulsion repräsentiert.

## Claims

1. Composition based on polyglycosides which comprises 30 to 65% by weight of at least one fatty alcohol of formula ROH, where R is a saturated or unsaturated, straight-chained or branched aliphatic radical or unsaturated aliphatic radical having 1 to 4 ethylenically unsaturated bonds and having 12 to 22 carbon atoms, the remainder being selected, apart from impurities, from:
- a mixture of polyglycosides containing 35 to 75% by weight, based on the polyglycosides, of polyhexosides of the formula:
R¹O(H)ₙ₁
wherein R¹ is a saturated or unsaturated, straight-chained or branched aliphatic radical or unsaturated aliphatic radical having 1 to 4 ethylenically unsaturated bonds and with 12 to 22 carbon atoms, H is the radical of a hexose, n1 is between 1 and 5; and 25 to 65% by weight, based on the polyglycosides, of at least one polypentoside of formula:
R²O(P) ₙ₂
wherein R² is a saturated or unsaturated, straight-chained or branched aliphatic radical or unsaturated aliphatic radical having 1 to 4 ethylenically unsaturated bonds, and having 12 to 22 carbon atoms, P is the radical of a pentose selected from arabinose and xylose, n2 is between 1 and 5;
- and a mixture of polyglycosides of formula:
R³O(G₁)ₐ(G₂)_{b}(G₃)_{c}(G₄)_{d}(G₅)ₑ
wherein R³ is a saturated or unsaturated, straight-chained or branched aliphatic radical or unsaturated aliphatic radical having 1 to 4 ethylenically unsaturated bonds and having 12 to 22 carbon atoms, G₁, G₂, G₃, G₄ and G₅ independently of one another are residues of an ose selected from the hexoses and pentoses, the latter being selected from arabinose and xylose, the hexoses representing 35 to 75% by weight of all the residues of oses G₁, G₂, G₃, G₄ and G₅ and the pentoses representing 25 to 75% by weight of all the residues of oses G₁, G₂, G₃, G₄ and G₅; a, b, c, d and e being equal to 0 or 1, and the sum of a, b, c, d and e being at least 1.

2. Composition according to claim 1, wherein the polyhexosides or hexose residues are made up, respectively, of at least 80% of polyglucosides or glucose residues.

3. Composition according to claim 1 which comprises polyglycosides wherein the R¹ and R² radicals or the radical R³ are identical to the radical R of the fatty alcohol.

4. Composition according to claim 1, wherein the fatty alcohol comprises 14 to 22 carbon atoms.

5. Composition according to claim 4, wherein the fatty alcohol is a mixture of alcohols having 16 to 18 carbon atoms.

6. Composition according to claim 1 which comprises 40 to 60% of fatty alcohol and preferably 52 to 57% of fatty alcohol.

7. Composition according to claim 1 which comprises a mixture of 45 to 70% by weight, based on the polyglycosides, of polyhexosides of formula R¹O(H)ₙ₁ and 30 to 55% by weight of polypentosides of formula R²O(P)ₙ₂.

8. Composition according to claim 1 which comprises polyglycosides of formula:
R³O(G₁) ₐ (G₂) _{b} (G₃) _{c} (G₄) _{d} (G₅)ₑ
wherein the hexose residues represent 45 to 70% by weight of all the ose residues G₁, G₂, G₃, G₄ and G₅ and the pentose residues constitute 30 to 55% by weight of all the ose residues G₁, G₂, G₃, G₄ and G₅.

9. Composition according to claim 1, wherein the polyglycosides are derived from sugar syrups obtained by hydrolysing by-products of vegetable origin rich in starch and hemicellulose.

10. Composition according to claim 1, wherein the polyglycosides are derived from wheat bran, mixtures of starch and wheat bran, maize bran, maize pomace or mixtures thereof.

11. Process for preparing an emulsion comprising contacting:
a) 2 to 60% by weight of an oily phase;
b) 1 to 10% by weight of a composition based on polyglycosides which comprises 30 to 65% by weight of at least one fatty alcohol of formula ROH, where R is a saturated or unsaturated, straight-chained or branched aliphatic radical or unsaturated aliphatic radical having 1 to 4 ethylenically. unsaturated bonds and having 12 to 22 carbon atoms, the remainder being selected, apart from impurities, from:
- a mixture of polyglycosides containing 35 to 75% by weight, based on the polyglycosides, of polyhexosides of the formula:
R¹O(H)ₙ₁
wherein R¹ is a saturated or unsaturated,straight-chained or branched aliphatic radical or unsaturated aliphatic radical having 1 to 4 ethylenically unsaturated bonds and with 12 to 22 carbon atoms, H is the radical of a hexose, n1 is between 1 and 5; and 25 to 65% by weight, based on the polyglycosides, of at least one polypentoside of formula:
R²O(P)ₙ₂
wherein R² is a saturated or unsaturated, straight-chained or branched aliphatic radical or unsaturated aliphatic radical having 1 to 4 ethylenically unsaturated bonds, and having 12 to 22 carbon atoms, P is the radical of a pentose selected from arabinose and xylose, n2 is between 1 and 5;
- and a mixture of polyglycosides of formula:
R³O(G₁)ₐ(G₂)_{b}(G₃)_{c}(G₄)_{d}(G₅)ₑ
wherein R³ is a saturated or unsaturated, straight-chained or branched aliphatic radical or unsaturated aliphatic radical having 1 to 4 ethylenically unsaturated bonds and having 12 to 22 carbon atoms, G₁, G₂, G₃, G₄ and G₅ independently of one another are residues of an ose selected from the hexoses and pentoses, the latter being selected from arabinose and xylose, the hexoses representing 35 to 75% by weight of all the residues of oses G₁, G₂, G₃, G₄ and G₅ and the pentoses representing 25 to 75% by weight of all the residues of oses G₁, G₂, G₃, G₄ and G₅; a, b, c, d and e being equal to 0 or 1, and the sum of a, b, c, d and e being at least 1;
c) the residues selected from among an aqueous phase or an aqueous phase to which has been added an emulsifying composition other than the one specified in b).

12. Process according to claim 11, wherein the oily phase of the emulsion contains at least 50% by weight, based on the total weight of the oily phase, of plant oils containing at least 40% of compounds selected from the linolenic acid triglycerides and and the silicon oils.

13. Process according to claim 11, wherein the oily phase represents 10 to 40% by weight based on the total weight of the emulsion.

14. Process according to claim 12, wherein composition b) represents 3 to 4% by weight of the total weight of the emulsion.
